(19) Europäisches Patentamt
European Patent Office
Office européen des brevets

(11) **EP 4 748 318 A1**

(12) **EUROPEAN PATENT APPLICATION**

(43) Date of publication:
27.05.2026 Bulletin 2026/22

(21) Application number: 25217629.2

(22) Date of filing: 21.11.2025

(51) International Patent Classification (IPC):
**A61B 8/08** (2006.01)    **A61B 8/12** (2006.01)
**A61B 8/00** (2006.01)

(52) Cooperative Patent Classification (CPC):
**A61B 8/085; A61B 8/12; A61B 8/4411;**
**A61B 8/461; A61B 8/463; A61B 8/464;**
**A61B 8/466; A61B 8/5207; A61B 8/523;**
**A61B 8/5238; A61B 8/565;** A61B 8/0841

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: 26.11.2024  CN 202411706133

(71) Applicant: **Carbon (Shenzhen) Medical Device Co,**
**Ltd.**
**Shenzhen, Guangdong 518101 (CN)**

(72) Inventors:
• **WANG, Shanshan**
**Shenzhen (CN)**
• **DENG, Yang**
**Shenzhen (CN)**
• **WU, Menglin**
**Shenzhen (CN)**

(74) Representative: **Metida**
**Gyneju str. 16**
**01109 Vilnius (LT)**

(54) **COMPUTER DEVICE, METHOD AND SYSTEM FOR IMPLEMENTING PUNCTURE GUIDANCE**

(57)    The present application relates to a computer device, a method and a system for implementing puncture guidance. The computer device comprises a memory and a processor. The memory stores a computer program. When the processor executes the computer program, the following steps are implemented: determining a safety treatment zone encompassing a lesion region of a target; determining puncture target points in the safety treatment zone; in response to a rotation operation of a ultrasound probe, updating a transverse plane image, mapping points, and an imaging area of a sagittal plane in real time in the first display area according to the rotation operation; when the target mapping point is located in the sagittal plane imaging area within the transverse plane image, a relative position of the target mapping point is displayed in the sagittal plane image in the second display area. In this way, an accuracy of puncture guidance is improved.

FIG.1

**Description**

TECHNICAL FIELD

The present application relates to a field of medical device technology, and in particular to a computer device, a method and a system for implementing puncture guidance.

BACKGROUND

Traditional puncture guidance devices or systems typically perform punctures on lesions or targets in the body under the monitoring and guidance of real-time acquired ultrasound images. The specific procedure involves avoiding important organs and larger blood vessels and nerves under the guidance of ultrasound, and accurately penetrating the interventional ablation device into the diseased tissue for treatment, or for aspirating or cutting out a small amount of cells or tissue for pathological examination.

Currently, operators are required to constantly and repeatedly observe the real-time relative position of the interventional ablation device and the target, as well as repeatedly observe ultrasound images to guide the puncture, which leads to problems such as inaccurate positioning of the puncture target and low work efficiency. Therefore, the prior art has certain drawbacks and needs to be improved and developed.

SUMMARY

Based on this, it is necessary to provide a computer device and a system for implementing puncture guidance to address the above technical problems.

In the first aspect, the present application provides a computer device for implementing puncture guidance, comprising a memory and a processor, wherein the memory stores a computer program, and when the processor executes the computer program, the following steps are implemented:

determining a safety treatment zone encompassing a lesion region of a target;
determining a plurality of puncture target points in the safety treatment zone;
constructing a 3D image based on the lesion region of the target, the safety treatment zone, and a contour of the target;
registering a plurality of ultrasound images obtained in real time with the 3D image to obtain a registration transformation matrix and a registered and fused 3D image; wherein the plurality of ultrasound images is obtained by an ultrasound probe, and the registration transformation matrix is used to indicate a spatial conversion relationship between a 3D coordinate system corresponding to the 3D image and a 2D coordinate system corresponding to the ultrasound image;
in the first display area, displaying a transverse plane image in the registered and fused 3D image, mapping points that are projected from puncture points on a puncture plate onto the transverse plane image, and a sagittal plane imaging area within the transverse plane image; wherein each of the plurality of puncture targets corresponds to a target mapping point, the target mapping point indicates the mapping point closest in distance to the corresponding puncture target;
in response to a rotation operation of the ultrasound probe, updating the transverse plane image, the mapping points, and the sagittal plane imaging area in the first display area in real time according to the rotation operation;
when the target mapping point is located in the sagittal plane imaging area within the transverse plane image, a relative position of the target mapping point located in the sagittal plane imaging area within the transverse plane image is displayed in the sagittal plane image in the second display area; wherein each of the plurality of puncture targets corresponds to a target mapping point, the target mapping point indicates the mapping point closest in distance to the corresponding puncture target.

In one embodiment, the lesion region is a 2D lesion region; and determining the safety treatment zone encompassing the lesion region of the target comprises:

determining, in multiple frames of 2D images of the target, a 2D image containing the 2D lesion region, and obtaining a preset first distance;
in the 2D image containing the 2D lesion region, generating an initial safety zone encompassing the 2D lesion region based on the preset first distance;
when a contour of the initial safety zone extends beyond the contour of the target, replacing the contour of an exceeding portion of the initial safety zone with the contour of the target to obtain a plurality of 2D safety treatment zones;

generating a 3D safety treatment zone based on the plurality of 2D safety treatment zones.

In one embodiment, the lesion region is a 3D lesion region; and determining the safety treatment zone encompassing the lesion region of the target comprises:

obtaining a unit normal vector of each of contour points on a surface of the 3D lesion region, and a second distance that each of the contour points moves along the unit normal vector;
moving each of the contour points along the unit normal vector by the second distance to obtain target points after the contour points are moved;
based on the target points, obtaining the safety treatment zone encompassing the 3D lesion region of the target.

In one embodiment, obtaining the second distance that each of the contour points moves along the unit normal vector comprises:

presetting an initial movement distance for each of the contour points along the unit normal vector;
respectively determining a temporary point after each of the contour points moves along corresponding unit normal vector based on the initial movement distance, 3D coordinates of each of the contour points, and the unit normal vector of each of the contour points;
determining a target ray formed by the 3D coordinates of the temporary point and the unit normal vector of the contour point;
when the number of intersection points between the target ray and the contour of the target is even, adjusting the initial movement distance until the number of intersection points between the target ray and the contour of the target is odd, and obtaining the second distance corresponding to each of the contour points.

In one embodiment, determining the plurality of puncture target points in the safety treatment zone comprises:

determining a composite safety area for the lesion region of the target according to the safety treatment zone, and the composite safety area being a 2D area;
according to size information of the composite safety area, dividing the composite safety area into blocks to obtain a plurality of sub-areas, and obtaining an ablation distance of an interventional ablation device;
in a contour of each of the plurality of sub-areas, respectively determining any contour point of each of the plurality of sub-areas as a first puncture target point, and determining a next puncture target point according to the ablation distance and a position of the first puncture target point, until the plurality of puncture target points are evenly distributed throughout each of the plurality of sub-areas.

In one embodiment, in the contour of each of the plurality of sub-areas, respectively determining any contour point of each of the plurality of sub-areas as the first puncture target point, and determining the next puncture target point according to the ablation distance and the position of the first puncture target point, until the plurality of puncture target points are evenly distributed throughout each of the plurality of sub-areas, comprising:

in each of the plurality of sub-areas, determining any contour point of each of the plurality of sub-areas as the first puncture target point;
determining a candidate puncture target point in each of the plurality of sub-areas based on the position of the first puncture target point and the ablation distance; wherein a distance between the candidate puncture target point and the first puncture target point is less than or equal to the ablation distance;
when the candidate puncture target point coincides with an avoidance position of the target, adjusting the candidate puncture target point until the adjusted candidate puncture target point no longer coincides with the avoidance position, and determining adjusted candidate puncture target point as the next puncture target point.

In one embodiment, the processor being further configured to execute the computer program to:

when the ultrasound probe and the puncture plate are orthogonal, obtain sensor coordinates, a first matrix and a second matrix of a positioning sensor in a spatial coordinate system by a positioning sensor connected to the ultrasound probe; wherein the first matrix is a matrix determined according to a sensor coordinate transformation, and the second matrix is used to represent a first position transformation relationship between the positioning sensor and the ultrasound probe;
according to the sensor coordinates, the first matrix, and/or the second matrix, determine a second position conversion relationship between the 2D coordinate system and the spatial coordinate system corresponding to the positioning sensor connected to the ultrasound probe;

When the target mapping point is located in the sagittal plane imaging area within the transverse plane image, a relative position of the target mapping point located in the sagittal plane imaging area within the transverse plane image being displayed in the sagittal plane image in the second display area, comprises:

determining coordinate data of the target mapping point located in the sagittal plane imaging area in the 2D coordinate system according to the second position conversion relationship;
determining target coordinate data of the target mapping point located in the sagittal plane imaging area in the 3D coordinate system according to the coordinate data and the registration transformation matrix;
in the second display area, the relative position of the target mapping point located in the sagittal plane image within the transverse plane image being displayed in the sagittal plane imaging area according to the target coordinate data.

In one embodiment, according to the sensor coordinates, the first matrix, and/or the second matrix, determining the second position conversion relationship between the 2D coordinate system and the spatial coordinate system corresponding to the positioning sensor connected to the ultrasound probe comprises any one of the following methods:

a first method: obtaining a first coordinate of the target mapping point in the 2D coordinate system, and determining a second coordinate of the target mapping point in the spatial coordinate system based on the first matrix, the second matrix, and the first coordinate; and establishing a second position transformation relationship based on the first coordinate and the second coordinate;
a second method: obtaining vertex coordinates of vertices of the transverse plane image in the spatial coordinate system based on the first matrix, the second matrix and a size of the transverse plane image; and determining second coordinates of the target mapping point in the spatial coordinate system based on the first coordinates and the vertex coordinates; and establishing the second position transformation relationship based on the first coordinates and the second coordinates.

In one embodiment, determining the coordinate data of the target mapping point located in the sagittal plane imaging area in the 2D coordinate system according to the second position conversion relationship comprises:

updating the second matrix of the positioning sensor in the spatial coordinate system to a third matrix; wherein the third matrix is used to represent a third position conversion relationship between the positioning sensor and a rotated ultrasound probe;
based on the second coordinates of the target mapping point located in the sagittal plane imaging area and the third matrix, determining the coordinate data of the target mapping point located in the sagittal plane imaging area in the 2D coordinate system corresponding to the updated transverse plane image by using the second position transformation relationship.

In a second aspect, the present application also provides a puncture guidance system, comprising: a computer device as described above, an ultrasound probe, an interventional ablation device, a puncture plate and a navigation unit; and the navigation unit comprises a positioning sensor; the ultrasound probe, the interventional ablation device, and the navigation unit are all communicatively connected to the computer device.
In a third aspect, the present application further provides a method for implementing puncture guidance comprising: determining a safety treatment zone encompassing a lesion region of a target; determining a plurality of puncture target points in the safety treatment zone; constructing a 3D image based on the lesion region of the target, the safety treatment zone, and a contour of the target; registering a plurality of ultrasound images obtained in real time with the 3D image to obtain a registration transformation matrix and a registered and fused 3D image; wherein the plurality of ultrasound images is obtained by an ultrasound probe, and the registration transformation matrix is used to indicate a spatial conversion relationship between a 3D coordinate system corresponding to the 3D image and a 2D coordinate system corresponding to the ultrasound image; in the first display area, displaying a transverse plane image in the registered and fused 3D image, mapping points that are projected from puncture points on a puncture plate onto the transverse plane image, and a sagittal plane imaging area within the transverse plane image; wherein each of the plurality of puncture targets corresponds to a target mapping point, the target mapping point indicates the mapping point closest in distance to the corresponding puncture target; in response to a rotation operation of the ultrasound probe, updating the transverse plane image, the mapping points, and the sagittal plane imaging area in the first display area in real time according to the rotation operation; when the target mapping point is located in the sagittal plane imaging area within the transverse plane image, a relative position of the target mapping point located in the sagittal plane imaging area within the transverse plane image being displayed in the sagittal plane image in the second display area.

The computer device, method and system for implementing puncture guidance described above determine the safety treatment zone that encompasses the lesion region of the target; determine multiple puncture target points in the safety treatment zone. When the lesion is detected extending beyond the determined lesion region of the target, multiple puncture target points can be determined in the safety treatment zone that encompasses the lesion region to guide the interventional ablation device to perform puncture and/or ablation. Moreover, in response to the rotation operation of the ultrasound probe, information such as the transverse plane image, the mapping points, and the sagittal plane imaging area can be updated in real time during the rotation of the ultrasound probe. When the target mapping point coincides with the sagittal plane imaging area within the transverse plane image, the relative position of the target mapping point located in the sagittal plane image within the transverse plane image is displayed in the sagittal plane imaging area in the second display area; thereby achieving a same target mapping point being displayed simultaneously in the transverse plane image and the sagittal plane image. On the one hand, the puncture position of each puncture can be accurately determined, with relatively high accuracy and stability, reducing the occurrence of multiple punctures due to inaccurate puncture positions; on the other hand, there is no need to repeatedly move the ultrasound probe to observe the puncture position for each puncture. After a last puncture, the ultrasound probe can be directly rotated to the next target mapping point for puncture, which simplifies the operation, saves time and improves puncture efficiency.

BRIEF DESCRIPTION OF THE DRAWINGS

FIG.1 is a schematic diagram showing a puncture guide system according to an exemplary embodiment.

FIG.2 is a flow chart showing a computer device for implementing puncture guidance according to an exemplary embodiment.

FIG.3 is a schematic diagram showing a transverse plane image according to an exemplary embodiment.

FIG.4 is a schematic diagram showing a transverse plane image according to an exemplary embodiment.

FIG.5 is a schematic diagram showing a transverse plane image and a sagittal plane image according to an exemplary embodiment.

FIG.6 is a schematic diagram showing an initial safety zone before contour replacement, according to an exemplary embodiment.

FIG.7 is a schematic diagram showing an initial safety zone after contour replacement , according to an exemplary embodiment.

FIG.8 is a schematic diagram showing overlapping areas according to an exemplary embodiment;

FIG.9 is a schematic diagram showing a puncture plate and an ultrasound probe in an orthogonal configuration, according to an exemplary embodiment.

FIG.10 is a schematic diagram showing a puncture plate and an ultrasound probe in a non-orthogonal configuration, according to an exemplary embodiment.

FIG.11 is a schematic diagram showing vertices on a transverse plane image according to an exemplary embodiment.

FIG.12 is a flow chart of a process for implementing puncture guidance by a computer device, according to an exemplary embodiment.

Fig.13 is a diagram of the internal structure of a computer device, according to an exemplary embodiment.

DESCRIPTION OF THE EMBODIMENTS

In order to make the objects, technical solutions, and advantages of the present disclosure clearer, the following detailed description is provided in conjunction with the accompanying drawings and embodiments. It should be understood that the specific embodiments described herein are intended to explain the present disclosure and are not meant to limit it.

The terms "first", "second" and "third" in the embodiments of the present application are used for descriptive purposes only and are not to be understood as indicating or implying relative importance or implicitly indicating the number of technical features indicated. Thus, a feature defined as "first", "second" and "third" may explicitly or implicitly include at least one of the features. In the description of the present application, "at least one" is used to indicate one or more; "plurality" means at least two, such as two, three, etc., unless otherwise clearly and specifically defined. In addition, the terms "including" and "having" and any variations thereof are intended to cover non-exclusive inclusions. For example, a process, method, apparatus, product or device comprising a series of steps or units is not limited to the listed steps or units, but may optionally also include steps or units that are not listed, or may optionally also include other steps or units inherent to these processes, methods, products or devices.

In this specification, references to "embodiments" herein mean that a particular feature, structure, or characteristic described in connection with the embodiments may be included in at least one embodiment of the present application. The appearance of the phrase in various places in the specification does not necessarily refer to the same embodiment, nor does it constitute an independent or alternative embodiment that is mutually exclusive of other embodiments. Those skilled in the art will explicitly and implicitly appreciate that the embodiments described herein can be incorporated in combination with other embodiments.

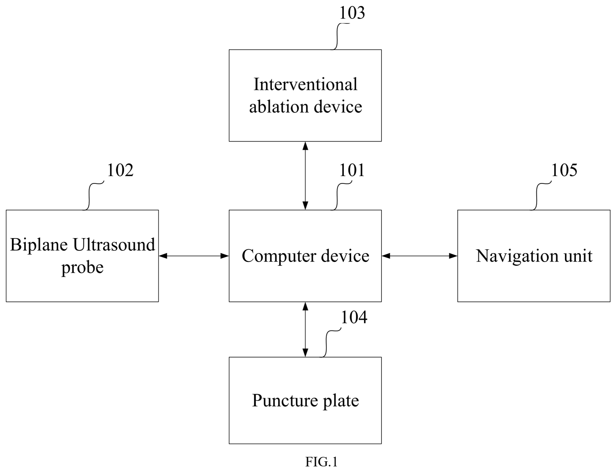

An embodiment of the present application provides a puncture guidance system. As shown in FIG.1, the puncture guidance system includes a computer device 101, an ultrasound probe 102, an interventional ablation device 103, a puncture plate 104, and a navigation unit 105. The navigation unit 105 includes a positioning sensor. The ultrasound probe 102, the interventional ablation device 103, the puncture plate 104, and the navigation unit 105 are all communicatively connected to the computer device 101. The computer device 101 can be used to implement the steps of guiding puncture of a target in any embodiment of the present application.

In the embodiments of the present application, the computer device may be any type of mobile terminal or fixed terminal. A terminal may be a device that provides voice and/or data connectivity to a user. For example, the terminal may be an Internet of Things (IoT) terminal, such as a sensor device, a mobile phone or so-called "cellular" phone, and a computer with the IoT terminal. For example, the terminal may be a fixed, portable, pocket-sized, handheld, computer-embedded, or vehicle-mounted device.

In some embodiments, a communication connection can include both wired and wireless connections. For example, the ultrasound probe 102, the interventional ablation device 103, the puncture plate 104, and the navigation unit 105 are all connected to the computer device 101 via a physical medium through a wired connection. Data transmission is carried out over physical lines, which offers enhanced stability and speed. Alternatively, the ultrasound probe 102, the interventional ablation device 103, the puncture plate 104, and the navigation unit 105 are all wirelessly connected to the computer device 101 via wireless technology. Data transmission is not limited by the physical lines, which provides greater flexibility.

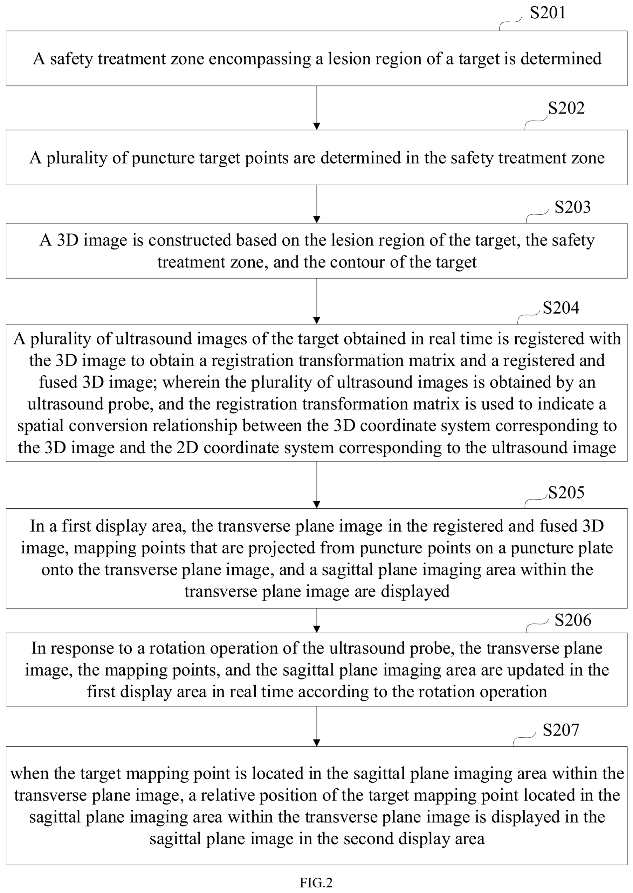

In some embodiments, as shown in FIG.2, a computer device for implementing puncture guidance is provided. The computer device includes a memory and a processor. The memory stores computer programs, and when the processor executes the computer programs, the following steps are implemented.

Step S201: a safety treatment zone encompassing a lesion region of a target is determined.

In the embodiment of the present application, the target refers to an object that may develop a lesion, and the target includes but is not limited to at least one of an organ, a tissue, or an anatomical structure.

In the embodiment of the present application, the lesion indicates a site where local pathological changes occur in an organism. The lesion region indicates the area where the lesion is located in the medical image sequence corresponding to the target acquired by the computer device.

For example, the lesion region can be obtained by inputting the medical image sequence into a segmentation model (e.g., a convolutional neural network model) to perform lesion region segmentation. Alternatively, the lesion region can be manually outlined in the medical image sequence. The method for dividing the lesion region is not limited herein.

In the embodiment of the present application, the safety treatment zone can be determined by an identification result of the lesion region. For example, based on the identification result of the lesion region, an area surrounding the lesion region in space is generated as the safety treatment zone.

Step S202: a plurality of puncture target points are determined in the safety treatment zone.

In some embodiments, the location of the plurality of puncture target points can be determined based on an ablation distance of the interventional ablation device, or any contour point of the safety treatment zone can be used as the puncture target point. For example, the computer device determines the plurality of puncture target points from contour of the safety treatment zone based on the ablation distance of the ablation needle.

Step S203: a 3D image is constructed based on the lesion region of the target, the safety treatment zone, and the contour of the target.

In the embodiment of the present application, the construction of the 3D image can be performed after the contour of the target, the contour of the lesion region, and the safety treatment zone are obtained. After obtaining the contour of the target, the contour of the lesion region, and the safety treatment zone in each frame of 2D (2D) slices, 3D volume data is constructed from the 2D contour points on each frame of 2D slices to obtain a 3D image. Each voxel represents a point in the 3D volume data, and the voxel contains a density value or a label value to indicate whether the point belongs to the target or the lesion. For gaps existing between 2D slices, the Marching Cubes algorithm can be utilized for interpolation processing to ensure the continuity and integrity of the 3D volume data. A DICOM or NIFTI file contains information such as image's direction matrix, pixel spacing, slice thickness, and origin position. This information is used to transform image coordinates into 3D coordinates in physical space, enabling the 3D image visualization.

Step S204: the plurality of ultrasound images obtained in real time is registered with the 3D image to obtain a registration transformation matrix and a registered and fused 3D image; wherein the plurality of ultrasound images is obtained by an ultrasound probe, and the registration transformation matrix is used to indicate a spatial conversion relationship between the 3D coordinate system corresponding to the 3D image and the 2D coordinate system corresponding to the ultrasound image.

In some embodiments, a detailed process of registering the real-time acquired ultrasound image of the target with the 3D image to obtain a registration transformation matrix is as follows: extracting feature points or key points from the ultrasound image and the 3D image, identifying corresponding points in a ultrasound point cloud and a 3D image point cloud; for each point in the ultrasound point cloud, calculating its nearest neighbor in the 3D image point cloud, and determining a point pair that minimizes the Euclidean distance; calculating a centroid of the ultrasound point cloud and the 3D image point cloud of a matching point set; using the centroid of the matching point set to calculate an optimal rotation matrix and a translation vector, and applying the matrix and the translation vector to the ultrasound point cloud to perform a rigid transformation to generate a new point set; calculating an average distance between a transformed ultrasound point cloud and the 3D image point cloud. The registration effect of current iteration is evaluated by calculating an average Euclidean distance between corresponding point clouds, and the iteration is determined to be complete based on the average Euclidean distance; and using a non-rigid registration algorithm to further optimize the registration results for a more accurate registration result. The finely registered ultrasound image and the 3D image are then fused to generate the registered 3D image.

In the embodiment of the present application, the ultrasound probe is used to obtain transverse plane (tra) images and/or sagittal plane (sag) images and display the images on a screen in real time. The ultrasound probe may include, but is not limited to, a single-plane ultrasound probe and a dual-plane ultrasound probe.

By way of example, the ultrasound probe may be a transrectal biplane endoluminal probe.

In the embodiment of the present application, a transverse plane (tra), also known as a horizontal plane or an axial plane, is used to indicate a cross-section parallel to the horizontal plane and perpendicular to a vertical axis when the human body is standing upright. In the medical imaging, the transverse plane image is obtained by collecting data parallel to the transverse plane during scanning.

In the embodiment of the present application, a sagittal plane (sag), also known as the longitudinal or lateral plane, is used to indicate a cross-section that passes through a sagittal axis and is perpendicular to the coronal and horizontal planes. The sagittal plane divides the human body into two symmetrical parts. In medical imaging, a sagittal plane image corresponds to a longitudinal section along the anterior-posterior direction of the human body, demonstrating the symmetrical structures on both sides of the body and their relative positional relationships.

In some embodiments, when the ultrasound probe is the dual-plane ultrasound probe, the computer device can transmit high-frequency sound waves through the ultrasound probe. As these sound waves propagate through human tissue, they encounter different interfaces (such as tissues, organs, and blood vessels), causing reflection, refraction, and scattering. The ultrasound probe receives these reflected sound wave signals and converts them into electrical signals, and after computer processing, images are obtained in two different directions, that is, transverse plane images and sagittal plane images of the target are simultaneously acquired.

In some embodiments, when the ultrasound probe is the single-plane ultrasound probe, a plurality of transverse plane images of the target can be acquired, and all the transverse plane images are superimposed. The superimposed transverse plane images are reconstructed using multi-plane reconstruction technology to obtain a 3D model of the target, and the sagittal plane images are obtained by slicing the 3D model.

Step S205: in a first display area, the transverse plane image in the registered and fused 3D image, mapping points that are projected from puncture points on a puncture plate onto the transverse plane image, and a sagittal plane imaging area are displayed.

In an embodiment of the present application, the puncture plate can be a positioning guide plate used to assist in puncture surgery. The puncture plate can be provided with a color band and a plurality of puncture points (puncture holes) arranged at fixed intervals. The color band is located between two adjacent puncture points and can be set to different colors. Medical personnel can determine the location of the puncture point simply by observing the color band, thereby more intuitively, conveniently, and accurately finding the puncture point corresponding to a target mapping point.

In some embodiments, the computer device can project the puncture point on the puncture plate onto the transverse plane image based on a mapping relationship between the transverse plane image and the puncture plate. The mapping relationship indicates a mapping relationship between the coordinate data of the transverse plane image and the coordinate data of the puncture point on the puncture plate. The computer device determines the mapping point closest to the puncture target point as the target mapping point. The puncture target point indicates the target puncture point in the safety treatment zone on the target determined by the computer device before registration. The target mapping point is used for puncture and/or ablation by the interventional ablation device.

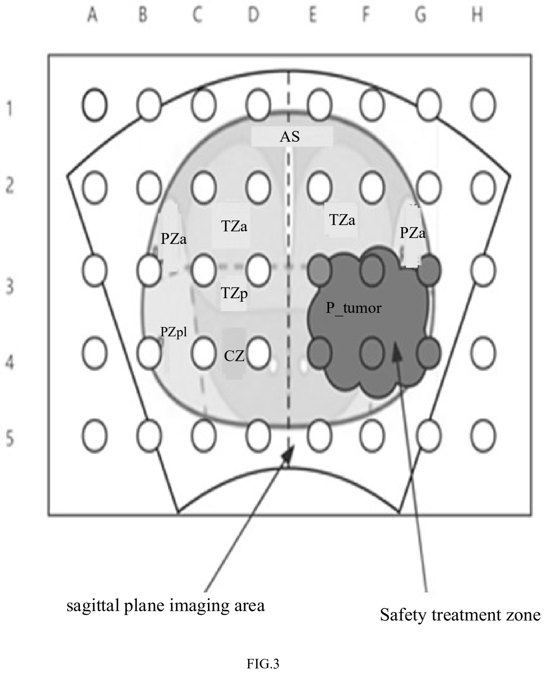

In some embodiments, the computer device projects the puncture points of the puncture plate onto the transverse plane image, thereby determining the mapping points within the safety treatment zone. The puncture points on the puncture plate exhibit a one-to-one correspondence with the mapping points in the transverse plane image. For example, when the ultrasound probe and the puncture plate are orthogonal, a schematic diagram of the projected transverse plane image is shown in FIG.3. The transverse plane image in FIG.3 includes an anterior fibromuscular stroma (AS), a central zone (CZ), an anterior peripheral zone (PZa), a posterior peripheral zone (PZpl), an anterior transitional zone (TZa), a posterior transitional zone (TZp), and the safety treatment zone (P_tumor) of the target.

In some embodiments, the imaging area representing the sagittal plane in the transverse plane image is a vertical centerline (symmetry axis) in the transverse plane image represents the sagittal plane imaging area within the transverse plane image. The safety treatment zone in FIG.3 includes 6 target mapping points, and none of the target mapping points is located on the vertical centerline.

Step S206: in response to a rotation operation of the ultrasound probe, the transverse plane image, the mapping points, and the sagittal plane imaging area are updated in the first display area in real time according to the rotation operation.

In some embodiments, when the target mapping point of the puncture is not located in the sagittal plane imaging area in the first display area, the ultrasound probe is rotated until the target mapping point of the puncture is located in the sagittal plane imaging area. During the rotation process, the computer device can scan and display the first display area in real time using the ultrasound probe. The first display area updates the transverse plane image, the mapping point, and the sagittal plane imaging area in real time based on the rotation operation, and the updated transverse plane image, the mapping point, and the sagittal plane imaging area are displayed in the first display area.

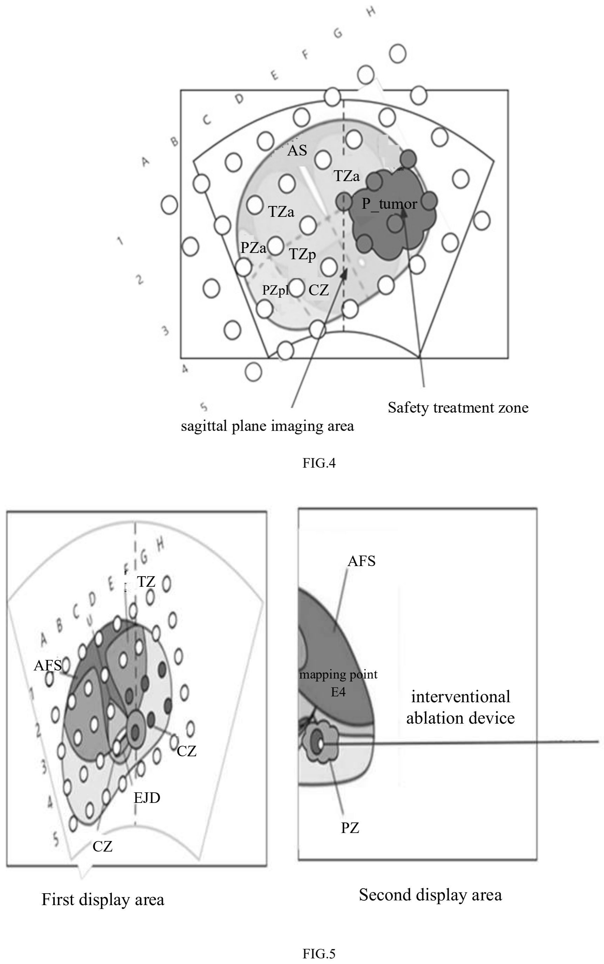

For example, as shown in FIG.3, which shows a transverse plane image before the ultrasound probe is rotated, the ultrasound probe and the puncture plate are orthogonal. Assuming that the target mapping point for current puncture is E3, E3 is located to the right side of the sagittal plane imaging area in the transverse plane image. As shown in FIG.4, which shows a schematic diagram of a transverse plane image after the ultrasound probe is rotated, the ultrasound probe and the puncture plate are non-orthogonal, and E3 is located in the sagittal plane imaging area in the transverse plane image. The transverse plane image in FIG.4 includes the target's anterior fibromuscular stroma (AS), central zone (CZ), anterior peripheral zone (PZa), posterior peripheral zone (PZpl), anterior transitional zone (TZa), posterior transitional zone (TZp), and safety treatment zone (P_tumor).

Step S207: when the target mapping point is located in the sagittal plane imaging area within the transverse plane image, a relative position of the target mapping point located in the sagittal plane imaging area within the transverse plane image is displayed in the sagittal plane image in the second display area; wherein each of the plurality of puncture targets corresponds to a target mapping point, the target mapping point indicates the mapping point closest in distance to the corresponding puncture target.

In some embodiments, as shown in FIG.5, the target is a prostate. FIG.5 illustrates a schematic diagram of a transverse plane image and a sagittal plane image of the prostate. FIG.5 includes a first display area and a second display area. The first display area is used to display the transverse plane image, and the second display area is used to display the sagittal plane image. The transverse plane image in the first display area includes an anterior fibromuscular layer (AFS), a transition zone (TZ), a central zone (CZ), and an ejaculatory duct (EJD) of the prostate. The sagittal plane image in the second display area includes the anterior fibromuscular layer (AFS) and a peripheral zone (PZ) of the prostate. When the target mapping point is located in the sagittal plane imaging area within the transverse plane image, a relative position of the target mapping point located in the sagittal plane imaging area within the transverse plane image is displayed in the sagittal plane image in the second display area. The ultrasound probe is roated until the target mapping point of the current puncture is located in the sagittal plane imaging area of the transverse plane image, thereby simultaneously displaying the target mapping point of the current puncture in real time in the sagittal plane image of the second display area.

The computer device and system for implementing puncture guidance described above determine the safety treatment zone that encompasses the lesion region of the target; determine a plurality of puncture target points in the safety treatment zone. When the lesion extends beyond the determined lesion area of the target object, the plurality of puncture target points can be determined in the safety treatment zone that encompasses the lesion region to guide the interventional ablation device to perform puncture and/ or ablation. Furthermore, in response to the rotation operation of the ultrasound probe, information such as the transverse plane image, mapping points, and sagittal plane imaging area can be updated in real time during the rotation of the ultrasound probe. When the target mapping point coincides with the sagittal plane imaging area within the transverse plane image, the relative position of the target mapping point located in the sagittal plane imaging area within the transverse plane image can be displayed in the second display area; thereby achieving the same target mapping point being displayed simultaneously in the transverse plane image and the sagittal plane image. On the one hand, the puncture position of each puncture can be accurately determined, with relatively high accuracy and stability, reducing the occurrence of multiple punctures due to inaccurate puncture positions; on the other hand, there is no need to repeatedly move the ultrasound probe to observe the puncture position for each puncture. After the last puncture, the ultrasound probe can be directly rotated until the next target mapping point coincides with the sagittal plane imaging area within the transverse plane image, so as to perform the next puncture, which simplifies the operation, saves time and improves puncture efficiency.

In some embodiments, the lesion region is a 2D lesion region; determining the safety treatment zone encompassing the lesion region of the target includes:

determining, in the multiple fames of 2D images containing the target, a 2D image including a 2D lesion region, and obtaining a preset first distance;

in the 2D image including the 2D lesion region, generating an initial safety zone encompassing the 2D lesion region based on the first distance;

when the outline of the initial safety zone extends beyond the contour of the target, replacing the portion of the initial safety zone's contour that lies beyond with the contour of the target to obtain multiple 2D safety treatment zones;

generating a 3D safety treatment zone based on the multiple 2D safety treatment zones.

In the embodiment of the present application, the multiple fames of 2D image containing the target refers to medical imaging data obtained by scanning the target from the sagittal plane, coronal plane and/or transverse plane of the target.

In some embodiments, a 2D image of the lesion region can be obtained using a segmentation model or manual identification. For example, multiple frames of 2D images containing the target are input into the segmentation model to identify and segment the lesion region, and then output a 2D image of the lesion region. In another example, each frame of the 2D image is examined one by one to determine the 2D image of the lesion region.

In some embodiments, the first distance refers to a distance by which each of the contour points in the 2D lesion area is to be offset. The first distance can be preset based on a standard distance between the 2D safety treatment zone and the 2D lesion region. For example, if the standard distance between the 2D safety treatment zone and the 2D lesion region is 2 mm to 8 mm, then any value between 2 mm and 8 mm can be used as the first distance. The first distance corresponding to each of the contour points can be the same or different. The first distance can also be determined based on the number of intersection points between a ray formed by the candidate point and a unit normal vector after each of the contour points in the 2D lesion region is moved, and the contour of the target. Specifically, the first distance is determined by: presetting an initial first distance for each of the contour points to be moved along the unit normal vector; determining the 2D coordinates of the candidate point after the contour point is moved along the unit normal vector based on the initial first distance, the 2D coordinates of the contour point, and the unit normal vector of the contour point; obtaining the ray formed by the 2D coordinates of the candidate point and the unit normal vector of the contour point; and if the number of intersection points between the ray and the contour of the target is even, adjusting the initial first distance until the number of intersection points between the ray and the contour of the target is odd, and determining the adjusted initial first distance as the first distance.

In some embodiments, generating the initial safety zone includes: moving each of the contour points of the 2D lesion region along corresponding unit normal vector in the opposite direction of the 2D lesion region based on the first distance corresponding to each of the contour points of the 2D lesion region, thereby obtaining a 2D target point after each of the contour points is moved; and then obtaining the initial safety zone based on the 2D target points. Techniques for obtaining the initial safety zone based on the 2D target points include, but are not limited to, a convex hull algorithm, an alpha shapes algorithm, and a Delaunay triangulation algorithm. For example, by using the convex hull algorithm, the minimum convex polygon generated by the 2D target points is smoothed to obtain the initial safety zone encompassing the 2D lesion region.

In some embodiments, the contour of the target can be determined by the segmentation model or manually identified. For example, multiple frames of 2D images containing the target are input into the segmentation model for segmentation, and the contour of the target in each frame of the 2D image can be identified.

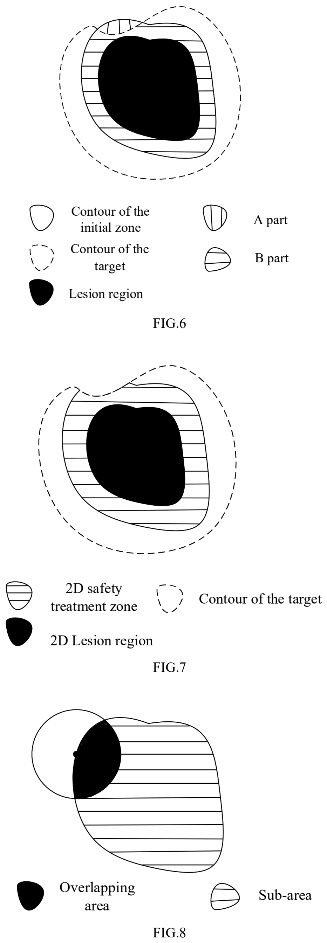

In some embodiments, the contour of the initial safety zone extends beyond the contour of the target, that is, the contour of the target is between the contour of the initial safety zone and the contour of the lesion region. Since the puncture target planning is based on the safety treatment zone, if the initial safety zone extends beyond the contour of the target, it will cause the puncture target point to be outside the contour of the target, which will cause damage to normal tissue outside the target. The contour of the exceeding part of the initial safety zone is replaced with the contour of the target. This can ensure that the planned puncture target points are all in the safety treatment zone, that is, it can ensure that the planned puncture target points are all inside the contour of the target and will not extend beyond the contour of the target. The schematic diagram before the initial safety zone is replaced is shown in FIG.6, part A is the part where the contour of the initial safety zone extends beyond the contour of the target, and part B is the part where the contour of the initial safety zone does not extend beyond the contour of the target. The schematic diagram after the initial safety zone is replaced is shown in FIG.7.

In some embodiments, methods for deriving a 3D safety treatment zone from multiple 2D safety treatment zones include, but are not limited to, interpolation techniques and surface reconstruction techniques. For example, 2D images of the 2D lesion regions are spatially aligned, the 2D contour points of the 2D safety treatment zone in each 2D image are converted into a 3D point cloud, depth information is assigned, and the 3D point cloud is subjected to Delaunay triangulation to generate a triangulated mesh. The 3D safety treatment zone is then derived based on the triangulated mesh.

Optionally, the computer device inputs multiple frames of 2D images containing the target into the segmentation model to perform lesion region identification and segmentation, determine the 2D image containing the lesion region, and obtain a preset first distance. Based on the first distance corresponding to each of the contour points of the 2D lesion region, the computer device moves each of the contour points of the 2D lesion region along the corresponding unit normal vector in the opposite direction of the 2D lesion region to obtain the 2D target point after each of the contour points is moved. The computer device uses the convex hull algorithm to generate the minimum convex polygon corresponding to the 2D image containing the 2D lesion region, and smooth the generated minimum convex polygon to obtain the initial safety zone that encompasses the 2D lesion region. When the contour of the initial safety zone extends beyond the contour of the target, the computer device replaces the contour of the exceeding part of the initial safety zone with the contour of the target to obtain multiple 2D safety treatment zones. The computer device generates the 3D safety treatment zone based on the multiple 2D safety treatment zones.

In one embodiment, generating the 3D safety treatment zone based on multiple 2D safety treatment zones includes:

determining a first frame image and a last frame image in the 2D images containing the 2D lesion region.

In the preceding n frames of 2D images adjacent to the first frame image, a region corresponding to the safety treatment zone of the first frame is generated, and the generated region is used as the safety treatment zone in the preceding n frames of 2D images.

In the succeeding n frames of 2D images adjacent to the last frame image, a region corresponding to the safety treatment zone of the last frame is generated, and the generated region is used as the safety treatment zone in the succeeding n frames of 2D images.

the 3D safety treatment zone is generated based on multiple 2D safety treatment zones, the safety treatment zone in the succeeding n frames of 2D images, and the safety treatment zone in the preceding n frames of 2D images.

In some embodiments, a single lesion in the target is presented in a 3D form, and the 2D image containing the 2D lesion region is obtained by continuously scanning the lesion. Therefore, the first 2D image frame in the 2D image containing the 2D lesion region is the first frame image, and the last 2D image frame is the last frame image. Since the safety treatment zone is the region that encompasses the lesion region but does not extend beyond the contour of the target, and the last 2D image frame in which the lesion appears is not the last 2D image frame in which the safety treatment zone appears, the safety treatment zone is also generated in the preceding n frames of 2D images before the first frame image and the succeeding n frames of 2D images after the last frame image.

By way of example, the value of n for the preceding n frames and succeeding n frames of 2D images can be determined using the following formula:

$$n = \mathrm{ceil}(SafetyDistance\,/\,SliceSpacing)$$

Wherein, ceil() function performs rounding up to the nearest integer, and SliceSpaceing represents the image spacing, or the distance between two image frames. SafeDistance represents an average spacing between safety zone contours, or the average distance between the lesion region and the safety treatment zone. Generally, the average spacing between safety zone contours is between 5 mm and 10 mm.

In some embodiments, a safety treatment zone in the preceding n frames of 2D images can be automatically generated based on the shape and the area of the safety treatment zone in the first frame. For example, based on the safety treatment zone in the first frame, a region with the same shape as the safety treatment zone in the first frame is generated in the preceding n frames of 2D images, but with a smaller area than the safety treatment zone in the first frame. The generated region is then the safety treatment zone in the preceding n frames of 2D images. The area of the safety treatment zone in the preceding n frames of 2D images gradually decreases. By generating the safety treatment zone in the preceding n frames of 2D images, the safety treatment zone can be supplemented in the 2D image where the lesion has disappeared, facilitating 3D reconstruction of the safety treatment zone.

In some embodiments, a safety treatment zone in the succeeding n frames of 2D images can be automatically generated based on the shape and area of the safety treatment zone in the last frame. For example, based on the safety treatment zone in the last frame, a region with the same shape as the safety treatment zone in the last frame, but smaller in area, is generated in the succeeding n frames of 2D images. The generated region serves as the safety treatment zone in the succeeding n frames of 2D images. The area of the safety treatment zone in the succeeding n frames of 2D images gradually decreases. By generating a safety treatment zone in the succeeding n frames of 2D images, the safety treatment zone can be supplemented in the 2D image where the lesion has disappeared, facilitating 3D reconstruction of the safety treatment zone.

Methods for generating the 3D safety treatment zone include, but are not limited to, an interpolation technique and a surface reconstruction technique. For example, the 2D images of each region containing the 2D lesion, the preceding n frames of 2D images, and the succeeding n frames of 2D images are spatially aligned. The 2D contour points of the safety treatment zone in each 2D image are converted into the 3D point cloud, assigned depth information, and the 3D point cloud is subjected to Delaunay triangulation to generate the triangulated mesh. Based on the triangulated mesh, the 3D safety treatment zone is obtained. Numerous clinical studies have demonstrated that positive lesions may exist approximately 5-10 mm beyond the lesion area visible on MRI. To achieve optimal therapeutic efficacy for the lesion area while minimizing damage to healthy organs and tissues, the planning of a safety treatment zone can enhance the effectiveness of the ablation. Although needle placement planning is ultimately performed within a 2D composite safety area, needle placement planning based on a 3D safety treatment zone helps determine the optimal puncture target point, thereby avoiding sensitive structures such as blood vessels and nerves and reducing the risk of complications.

Optionally, the computer device determines the first frame of the 2D image containing the lesion region as the first frame image, and determines the last frame of the 2D image containing the lesion region as the last frame image. The computer device generates safety treatment zones in the preceding n frames of the 2D image adjacent to the first frame image according to the shape and area of the safety treatment zone in the first frame image. The computer device generates safety treatment zones in the succeeding n frames of the 2D image adjacent to the last frame image according to the shape and area of the safety treatment zone in the last frame image. The computer device aligns the 2D images containing the 2D lesion region, the preceding n frames of the 2D image, and the succeeding n frames of the 2D image in space, converts the 2D contour points of the safety treatment zone in each frame of the 2D image into the 3D point cloud, and assigns depth information, and performs Delaunay triangulation on the 3D point cloud, and generates the triangulated mesh, and obtains the 3D safety treatment zone based on the triangulated mesh.

In the embodiment, by replacing the contour of the exceeding portion in the initial safety zone with the contour of the target, it is ensured that the planned puncture target points are all within the safety treatment zone, that is, it is ensured that the planned puncture target points are all within the contour of the target and do not extend beyond the contour of the target.

In some embodiments, the lesion region is the 3D lesion region; and determining the safety treatment zone encompassing the lesion region of the target includes:

obtaining a unit normal vector of each of the contour points on the surface of the 3D lesion region, and a second distance that each of the contour points moves along the unit normal vector;

moving each of the contour points along the unit normal vector by the second distance to obtain target points after the contour points are moved;

based on the target points, obtaining the safety treatment zone encompassing the 3D lesion region of the target.

In some embodiments, the second distance is a distance that the contour point on the surface of the 3D lesion region is to be moved from its current position. The coordinates of the 3D target point after the contour point moves along the unit normal vector according to the second distance can be calculated according to formulas: $x' = x + t \cdot a'$, $y' = y + t \cdot b'$, $z' = z + t \cdot c'$, wherein $(x', y', z')$ are the coordinates of the 3D target point after the contour point moves, $(x, y, z)$ are the 3D coordinates before the contour point moves, $(a', b', c')$ are the unit normal vectors of the contour point, and t is the second distance. After any contour point in the lesion region moves, the contour of the lesion region does not change. The 3D coordinates of the contour point before movement can be obtained by converting the 2D coordinates of the contour point using a space transformation matrix. The moving direction of each of the contour points on the surface of the 3D lesion region is opposite to the direction of the 3D lesion region.

In some embodiments, the unit normal vector of the contour point is a normalized normal vector. Methods for determining the unit normal vector of each of the contour points include, but are not limited to, surface gradient calculation methods and least squares calculation methods. For example, the computer device determines a contour point *lesionPtPhy$_{m,n}$*, where *lesionPtPhy$_{m,n}$* represents the n-th contour point of the m-th plane of the 3D lesion region. The m-th plane of the 3D lesion region can be understood as the m-th frame image obtained after slicing the 3D lesion region. The computer device searches p planes forward and backward from the m-th plane respectively, and in the searched 2p +1 planes, with the current point as the center, searches for a total of 2q+1 adjacent points forward and backward, wherein current point on each plane is determined based on the search starting point from its adjacent plane, thus ensuring the rationality of each current point selection and avoiding sudden jumps or discontinuities; a total of (2p+1)(2q+1) points are searched $\{lesionPtPhy_{x,y} \mid x-p < x < x+p, y-q < y < y+q\}$, and the computer device uses the least squares method to solve the unknown coefficients *a,b,c,d,e,f* of the surface $z = \dfrac{ax^2 + by^2 + cx + dy + e}{f}$, where x represents the x-th plane and y represents the y-th contour point on the x-th plane. A console obtains the normal vector

$$\left(Fx', Fy', Fz'\right) = \left(\frac{2ax+c}{f}, \frac{2bx+d}{f}, \frac{e}{f}\right)$$ of the contour points based on the solution results of each unknown coefficients, and the computer device normalizes the normal vector of the contour point to obtain the unit normal vector *norm$_{m,n}$* of the contour point.

In some embodiments, the 3D safety treatment zone may be generated by generating the triangulated mesh based on the position coordinates of each target point by the Delaunay triangulation algorithm, and constructing the 3D safety treatment zone based on the triangulated mesh.

Optionally, the computer device obtains, for each of the contour points on the surface of the 3D lesion region, a second distance to be moved from its current position, as well as the unit normal vector at each of the contour points. The computer device moves each of the contour points along the unit normal vector by the corresponding second distance to obtain a target point for each of the contour points after the movement. The computer device generates the triangulated mesh based on the position coordinates of each target point using the Delaunay triangulation algorithm, and constructs the 3D safety treatment zone based on the triangulated mesh.

In an embodiment of the present application, the contour point is moved along the unit normal vector according to the second distance, which can effectively expand the original lesion region to form a larger safety treatment zone whose shape corresponds to the lesion region. This ensures that when there are still positive parts outside the lesion region, the positive parts outside the lesion region can be punctured and the target points can be planned to perform ablation treatment on the positive parts outside the lesion region, thereby improving the effectiveness of ablation.

In some embodiments, obtaining the second distance that each of the contour points moves along the unit normal vector includes:

presetting an initial movement distance for each of the contour points along the unit normal vector;

respectively determining the 3D coordinates of a temporary point after each of the contour points moves along corresponding unit normal vector based on the initial movement distance, the 3D coordinates of each of the contour points, and the unit normal vector of each of the contour points;

determining a target ray formed by the 3D coordinates of the temporary point and the unit normal vector of the contour point;

when the number of intersection points between the target ray and the contour of the target is an even number, adjusting the initial movement distance until the number of intersection points between the target ray and the contour of the target is an odd number, thereby obtaining the second distance corresponding to each of the contour points.

In some embodiments, the initial movement distance can be preset based on the average spacing between safety zone contours. For example, when the average spacing between safety zone contours ranges from 5mm to 10mm, the initial movement distance can be preset to 10mm. Alternatively, the initial movement distance can be preset to any value between 5mm and 10mm. In general scenarios, the maximum value of the initial movement distance is consistent with the maximum value of the average spacing between safety zone contours. Each of the contour points corresponds to an initial movement distance, and the initial movement distance corresponding to each of the contour points can be the same or different.

In some embodiments, the 3D coordinates of each of the contour points can be obtained by the 2D coordinates of the contour point in the 2D image and the space transformation matrix. For example, the 3D coordinates of the temporary point after the contour point is moved along the unit normal vector can be calculated according to formulas: $x'_1 = x + t_1 \cdot a'$,

$y'_1 = y + t \cdot b'$, $z'_1 = z + t_1 \cdot c'$, where $(x'_1, y'_1, z'_1)$ represents the 3D coordinate of the temporary point after the contour point is moved, $(x, y, z)$ represents the 3D coordinate of the contour point before the movement, $(a', b', c')$ represents the unit normal vector of the contour point, $t_1$ represents the initial movement distance.

Exemplarily, the target ray composed of the 3D coordinates of the temporary point and the unit normal vector of the contour point can be expressed as $line_{m,n} = tempMarginPtPhy_{m,n} + i \times norm_{m,n}$, $i > 0$, wherein, $line_{m,n}$ represents the target ray corresponding to the contour point $tempMarginPtPhy_{m,n}$, and $lesionPtPhy_{m,n}$ represents the 3D coordinates of the temporary point after the contour point is moved $lesionPtPhy_{m,n}$, and $norm_{m,n}$ represents the unit normal vector of the contour point $lesionPtPhy_{m,n}$, and $lesionPtPhy_{m,n}$ represents the n-th contour point on the lesion region of the m-th plane in the 3D lesion region, and i is a non-negative real number parameter used to represent the distance moved from the temporary point along the unit normal vector of the contour point. When i=0, the point is exactly located at the temporary point; and when i >0, the point is located somewhere between the temporary point and the unit normal vector.

In some embodiments, the contour of the target refers to a 3D outline. The unit normal vector of the target point is always within the contour of the target. If the temporary point is outside the contour of the target, then the target ray formed by the temporary point and the unit normal vector of the target point must first enter the contour of the target and then pass through the contour of the target. In this case, the number of intersection points between the target ray and the contour of the target is an even number. If the temporary point is within the contour of the target, then the target ray formed by the temporary point and the unit normal vector directly passes through the contour of the target from the inside of the contour of the target. In this case, the number of intersection points between the target ray and the contour of the target is an odd number. When the interventional ablation device is used for ablation, an endpoint of the electrode of the interventional ablation device needs to be located within the safety treatment zone or the contour of the target. In this way, no damage is caused to other objects when the lesion is ablated. Therefore, when the number of intersection points between the target ray and the contour of the target is the even number, the initial distance needs to be adjusted until the number of intersection points between the target ray and the contour of the target is the odd number.

In some embodiments, the initial movement distance can be adjusted by changing a fixed value or an arbitrary value. For example, when the temporary point is determined to be outside the contour of the target by the number of intersection points, the initial movement distance can be adjusted by increasing/decreasing the fixed value or increasing/decreasing an arbitrary value based on the initial movement distance. The size of the fixed value and the arbitrary value can be set arbitrarily. For example, if the average spacing between safety zone contours is within 5-10mm, then the initial movement distance starts from 10mm and is adjusted in steps of 0.1. That is, when it is determined that the temporary point is outside the contour of the target, the current initial movement distance is reduced by 0.1 until the number of intersection points between the target ray and the contour of the target is the odd number. The adjusted initial movement distance is then the second distance.

Optionally, the computer device presets the initial movement distance for the contour point along the unit normal vector based on the average spacing between safety zone contours. Based on the initial movement distance, the 3D coordinates of the contour point, and the unit normal vector of the contour point, the computer device calculates the 3D coordinates of the temporary point after the contour point has moved along the unit normal vector. The computer device forms the target ray based on the 3D coordinates of the temporary point and the unit normal vector of the contour point, and determines the number of intersection points between the target ray and the contour of the target. When the number of intersection points is the even number, the computer device adjusts the initial movement distance of the contour point based on the preset fixed value or the arbitrary value until the number of intersection points between the target ray and the contour of the target is the odd number, and determines the adjusted initial movement distance as the second distance.

In the embodiment of the present application, when the number of intersection points between the target ray and the contour of the target is the even number, the initial movement distance is adjusted until the number of intersection points between the target ray and the contour of the target is the odd number. This ensures that the target point remains within the contour of the target after the contour point moves, so that the safety treatment zone generated based on the target point does not extend beyond the contour of the target, thereby ensuring that the planned puncture target points do not extend beyond the contour of the target, thereby not damaging other normal tissues during puncture and/or ablation.

In some embodiments, determining multiple puncture target points in the safety treatment zone includes:

determining a composite safety area for the lesion region of the target according to the safety treatment zone, wherein the composite safety area is a 2D area;

according to the size information of the composite safety area, dividing the composite safety area into blocks to obtain multiple sub-areas, and obtaining an ablation distance of the interventional ablation device;

in the contour of each of the sub-areas, respectively determining any contour point of the sub-area as the first puncture target point, and determining a next puncture target point according to the ablation distance and the position of the first puncture target point, until the puncture target points are evenly distributed throughout the sub-area.

In some embodiments, the computer device can project the 3D safety treatment zone directly into a 2D plane to obtain the composite safety area.

In one embodiment, determining the composite safety area for the lesion region of the target includes:

obtaining the 3D safety treatment zone, wherein the 3D safety treatment zone is a zone surrounding the lesion region of the target;

slicing the 3D safety treatment zone to obtain multiple 2D safety treatment zones;

projecting the multiple 2D safety treatment zones onto the same 2D plane to obtain the composite safety area of the target.

In the embodiments of the present application, the 3D safety treatment zone refers to an area encompassing a 3D lesion region. The 3D safety treatment zone can be derived from multiple 2D safety treatment zones. Methods for deriving the 3D safety treatment zone from multiple 2D safety treatment zones include, but are not limited to, interpolation techniques and surface reconstruction techniques. The 3D safety treatment zone can also be generated directly from target points obtained by moving contour points on the surface of the 3D lesion region.

In some embodiments, when slicing the 3D safety treatment zone, the computer device slices the area based on the slice spacing. The slice spacing refers to a distance between two adjacent 2D safety treatment zones. The slice spacing can be pre-set in the computer device or randomly assigned by the computer device.

In some embodiments, multiple 2D safety treatment zones are displayed in a 2D image including a 2D lesion region and a 2D target. When projecting, the multiple 2D safety treatment zones need to be extracted from the 2D image first and then projected into the 2D plane. The method for extracting the 2D safety treatment zone includes, but is not limited to, using a U-shaped network to extract. The image of the 2D safety treatment zone extracted by the U-shaped network can be a binary image, which can more intuitively distinguish the safety treatment zone and the non-safety treatment zone on the 2D image. It should be noted that since the composite safety area is the area obtained by projecting multiple 2D safety treatment zones onto the same 2D plane, the composite safety area can be understood as the area after taking the union of multiple 2D safety treatment zones.

Optionally, the computer device obtains the 3D safety treatment zone that encompasses the 3D lesion region. The computer device slices the 3D safety treatment zone according to a preset slice spacing to obtain multiple 2D safety treatment zones. The multiple 2D safety treatment zones are displayed in the 2D image that includes the 2D lesion region and the 2D target. The computer device uses the U-shaped network to extract the safety treatment zone in each frame of the 2D image and projects the extracted multiple 2D safety treatment zones onto the same 2D plane to obtain the composite safety area.

In the embodiment of the present application, the size information includes, but is not limited to, at least one of the area, perimeter and diameter of the composite safety area.

In some embodiments, the segmentation of the composite safety area according to size information may be performed based on the area of the composite safety area, or based on the perimeter of the composite safety area, or based on the diameter of the composite safety area. For example, if the total area of the composite safety area is S and it is predetermined to be segmented into multiple sub-areas of area s, then during segmentation, the computer device segments the composite safety area into multiple sub-areas of area s according to the area S of the composite safety area.

In the embodiments of the present application, an interventional ablation device is a medical apparatus that delivers energy to the lesion of the target via a catheter or other miniature instruments to destroy the lesion. The interventional ablation device includes, but is not limited to, at least one of a radiofrequency ablation device, a microwave ablation device, a cryoablation device, and a chemical ablation device.

In the embodiments of the present application, the ablation distance of the interventional ablation device indicates a maximum distance from the ablation probe or electrode tip of the interventional ablation device to which it can effectively generate an ablative effect capable of destroying the target lesion. The ablation distance of the interventional ablation device can be pre-stored in the computer device so that the computer device can promptly read the ablation distance of the interventional ablation device.

In some embodiments, obtaining the ablation distance of the interventional ablation device can be performed simultaneously with the division of the composite safety area, or after the division into multiple sub-areas, or when determining the composite safety area for the lesion region of the target.

Optionally, the computer device divides the composite safety area into blocks according to the area, or the perimeter, or the diameter of the composite safety area to obtain multiple sub-areas.

In some embodiments, the first puncture target point determined in each of the sub-areas does not coincide with any avoidance position of the target. The avoidance positions of the target include, but are not limited to, various critical parts and tissues of the target, for example, the locations of bones and blood vessels. The avoidance positions can be pre-entered into the computer device for easy access when compared with the puncture target points. The avoidance positions can be identified using static medical images acquired preoperatively. Static medical images may include, but are not limited to, X-ray imaging, Computerized Tomography (CT) imaging, and Nuclear Magnetic Resonance Imaging (MRI). By comparing the position of the first puncture target point with each avoidance position, it can be determined whether the first puncture target point coincides with the avoidance position, therefore preventing the planning of needle placement at the avoidance position, avoiding damage to critical structures or tissues of the target.

In some embodiments, the distance between the next puncture target point and the first puncture target point is less than or equal to the ablation distance of the interventional ablation device. This ensures that when the interventional ablation device performs puncture and/or ablation based on the puncture target point, the ablation effect produced by the ablation probe or electrode tip of the interventional ablation device can spread throughout the entire sub-area, that is, throughout the entire composite safety area, thereby improving the effectiveness of ablation.

In some embodiments, the position of the next puncture target point may be located on the contour of the sub-area or inside the sub-area.

In some embodiments, the puncture target planning of each of the sub-areas can be performed simultaneously, which can reduce the time required for puncture target planning of the composite safety area and improve the efficiency of puncture target planning, so that the composite safety area of the target can be quickly ablated, thereby improving the efficiency of ablation treatment.

Optionally, the computer device determines any contour point in the contour of each of the sub-areas that does not coincide with the avoidance position of the target as the first puncture target point. Based on the position of the first puncture target point and the ablation distance of the ablation device, the computer device determines a point in the sub-area whose distance from the first puncture target point is less than or equal to the ablation distance as the next puncture target point. In some embodiments, in the contour of each of the sub-areas, respectively determining any contour point of the sub-area as the first puncture target point, and determining a next puncture target point according to the ablation distance and the position of the first puncture target point, until the puncture target points are evenly distributed throughout the sub-area, includes:

in each of the sub-areas, respectively using any contour point of the sub-area as the first puncture target point;

determining a candidate puncture target point in the sub-area based on the position of the first puncture target point and the ablation distance; wherein the distance between the candidate puncture target point and the first puncture target point is less than or equal to the ablation distance;

when the candidate puncture target point coincides with the avoidance position of the target, adjusting the candidate puncture target point until the adjusted candidate puncture target point does not coincide with the avoidance position, and determining the adjusted candidate puncture target point as the next puncture target point.

In some embodiments, the first puncture target point determined in each of the sub-areas does not coincide with the avoidance position of the target. The candidate puncture target points can be determined from the contour of the sub-area or from within the sub-area. For example, a circle is drawn with the first puncture target point as the center and the ablation distance of the interventional ablation device as the radius. The candidate puncture target point can be determined in the area where the circle coincides with the sub-area. The overlapping area used to determine the candidate puncture target points is shown in FIG.8.

In some embodiments, the avoidance position refers to a position within the target where needle placement is prohibited. The avoidance position includes, but is not limited to, various important parts and tissues. By comparing the position of the candidate puncture target point with each avoidance position, it can be determined whether the candidate puncture target point coincides with the avoidance position. The adjusted puncture target point should also not coincide with the avoidance position, and the distance between the adjusted puncture target point and the first puncture target point should be less than or equal to the ablation distance of the interventional ablation device. For example, when the determined candidate puncture target point coincides with the avoidance position, the position that is closer to the first puncture target point and does not coincide with the avoidance position is determined as the position of the candidate puncture target point.

In some embodiments, when the candidate puncture target point does not coincide with the avoidance position, the candidate puncture target point can be directly determined as the next puncture target point. Furthermore, by the next puncture target point and the ablation distance of the interventional ablation device, a puncture target point can be determined again, until the puncture target points are evenly distributed throughout the entire sub-area and there are no overlapping puncture target points. For example, first, using the selected first puncture target point as the center and the ablation distance of the interventional ablation device as the radius, draw a circle A, and the intersection point a of circle A and the sub-area is the candidate puncture target point; then, using the obtained intersection point a as the center and the ablation distance of the interventional ablation device as the radius, draw a circle B, and identify the intersection point b of circle B and the sub-area; when the next intersection point b does not coincide with the avoidance position, the next intersection point b is the re-determined puncture target point, until the puncture target points are evenly distributed throughout the entire sub-area and there are no overlapping puncture target points.

Optionally, in each of the sub-areas, the computer device uses any contour point in the boundary of the sub-area as the first puncture target point, and determines a candidate puncture target point in the sub-area whose distance from the first puncture target point is less than or equal to the ablation distance. The computer device compares the candidate puncture target point with each pre-stored avoidance position. If the candidate puncture target point coincides with one of the avoidance positions, the computer device adjusts the candidate puncture target point, and if the adjusted candidate puncture target point does not coincide with any of the avoidance positions, the computer device determines the adjusted candidate puncture target point as the next puncture target point, wherein the distance between the adjusted candidate puncture target point and the first puncture target point is less than or equal to the ablation distance, and the replaced candidate puncture target point is also located in the sub-area. If the candidate puncture target point does not coincide with the avoidance position of the target, the computer device directly determines the candidate puncture target point as the next puncture target point.

In some embodiments, the computer device obtains tissue dielectric parameters of electrodes of the interventional ablation device in various targets. Tissue dielectric parameters are parameters that affect the ablation effect of the electrodes and include, but are not limited to, tissue conductivity and dielectric constant. The computer device uses a finite element analysis simulation software to calculate the electric field distribution under a current puncture target planning scheme to analyze the response of each target to the electric field and obtain simulation results. The current puncture target planning scheme includes the positions of each puncture target point determined based on the ablation distance, the first puncture target point, and the avoidance position. Based on the simulation results, the computer device adjusts the puncture target points to optimize the electric field distribution, obtaining a new puncture target planning scheme. The new puncture target planning scheme includes the adjusted positions of each puncture target point. The computer device adjusts the puncture target points based on the simulation results to optimize the electric field distribution by, for example, reallocating puncture target points, adding or reducing puncture target points. This ensures that the lesions in the target receive sufficient electric field strength while minimizing damage to normal tissue.

In the embodiment of the present application, by determining the composite safety area for the lesion region of the target, dividing the composite safety area into blocks according to the size information of the composite safety area to obtain multiple sub-areas, and obtaining the ablation distance of the interventional ablation device, the puncture target planning of each of the sub-areas can be carried out simultaneously, which can improve the efficiency of puncture target planning, so that the lesion region of the target can be quickly ablated, thereby improving the efficiency of ablation treatment. By taking any contour point of the sub-area as the first puncture target point in the contour of each of the sub-areas, and determining the next puncture target point according to the ablation distance and the position of the first puncture target point, until the puncture target points are evenly distributed throughout the sub-area, the efficiency of ablation treatment can be further improved, and the lesion region of the target can also be thoroughly ablated, thereby improving the effectiveness of ablation and reducing the occurrence of lesion ablation again due to incomplete ablation.

In some embodiments, when the processor executes the computer program to implement the following steps:

when the ultrasound probe and the puncture plate are orthogonal, the sensor coordinates, obtaining a first matrix and a second matrix of the positioning sensor in corresponding spatial coordinate system by a positioning sensor connected to the ultrasound probe; wherein the first matrix is a matrix determined according to the sensor coordinate transformation; the second matrix is used to represent a first position transformation relationship between the positioning sensor and the ultrasound probe;

according to the sensor coordinates, the first matrix, and/or the second matrix, determining a second position conversion relationship between the 2D coordinate system and the spatial coordinate system corresponding to the positioning sensor connected to the ultrasound probe;

when the target mapping point is located in the sagittal plane imaging area within the transverse plane image, a relative position of the target mapping point located in the sagittal plane imaging area within the transverse plane image being displayed in the sagittal plane image in the second display area, includes:

determining coordinate data of the target mapping point located in the sagittal plane imaging area in the 2D coordinate system according to the second position conversion relationship;

determining target coordinate data of the target mapping point located in the sagittal plane imaging area in the 3D coordinate system according to the coordinate data and the registration transformation matrix;

in the second display area, the relative position of the target mapping point located in the sagittal plane image within the transverse plane image being displayed in the sagittal plane imaging area according to the target coordinate data.

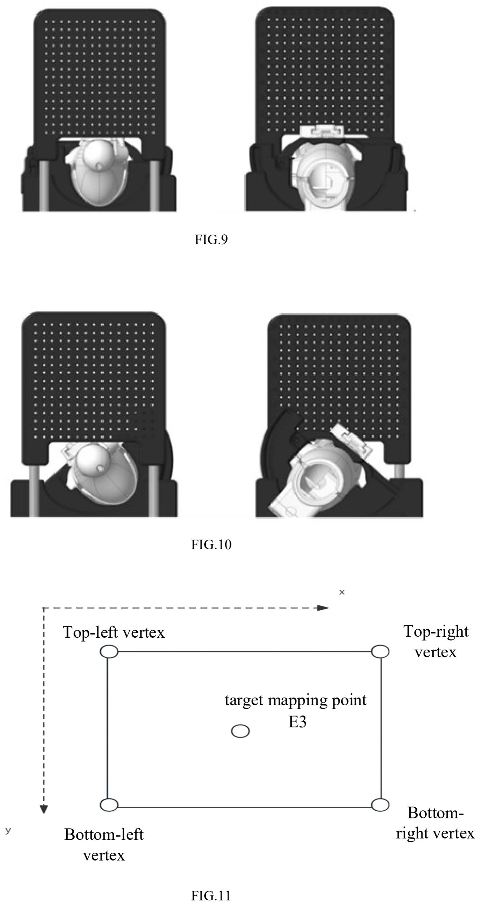

By way of example, as shown in FIG.9, FIG.9 is a schematic diagram showing that the puncture plate is orthogonal to the ultrasound probe.

By way of example, when the ultrasound probe is rotated, as shown in FIG.10, which is a display schematic diagram after the ultrasound probe is rotated, the ultrasound probe and the puncture plate are in a non-orthogonal state.

By way of example, the sensor coordinates may be: $T_{sensor} = (x, y, z)$. A calculation method for determining the first matrix is:
$$TM\_sensor = \begin{bmatrix} R_{sensor} & T_{sensor} \\ 0 & 1 \end{bmatrix}$$
; wherein, $R_{sensor}$ indicates the rotational posture matrix of the positioning sensor in the spatial coordinate system. The computer device may determine the second matrix TM_trans in advance by measurement and/or calibration.

In some embodiments, the positioning sensor can be an electromagnetic tracker, and the spatial coordinate system can be a magnetic field coordinate system. The electromagnetic tracker senses the magnetic field and can calculate the coordinate information of the puncture plate and/or guided ablation device within the magnetic field coordinate system. Alternatively, the positioning sensor can be an optical tracker, and the spatial coordinate system can be a 3D coordinate system. By attaching markers to a surgical instrument, the optical tracker illuminates the target with infrared light. The infrared light is then reflected from the markers and returned to the infrared sensor on the optical tracker. The intersection of the light rays can be used to triangulate the 3D coordinates of the markers on the target.

In the embodiment of the present application, the position conversion relationship may indicate the coordinate data conversion between two coordinate systems. For example, the second position conversion relationship may be a conversion from a 2D coordinate system to a spatial coordinate system, or from the spatial coordinate system to the 2D coordinate system.

By way of example, a method for determining the target coordinate data of a target mapping point in the 3D coordinate system is: $P_{E3\_3d\_new} = TM\_Reg \times P_{E3\_2d\_new}$; wherein, TM_Reg indicates the registration transformation matrix; and $P_{E3\_2d\_new}$ indicates coordinate data of the target mapping point in the 2D coordinate system; and $P_{E3\_3d\_new}$ indicates target coordinate data of the target mapping point in the 3D coordinate system.

In some embodiments, the computer device can update the coordinate data of any target mapping point in the 2D coordinate system in the updated transverse plane image in real time based on the second position conversion relationship, so as to accurately determine the coordinate data of the target mapping point located in the sagittal plane imaging area within the transverse plane image during the current puncture (i.e., the target mapping point of the current puncture) in the 3D coordinate system.

In the embodiment of the present application, by determining the second position conversion relationship between the 2D coordinate system and the spatial coordinate system corresponding to the positioning sensor connected to the ultrasound probe when the ultrasound probe is orthogonal to the puncture plate, the coordinate data of the 2D coordinate system of any target mapping point in the updated transverse plane image can be updated in real time according to the second position conversion relationship during the rotation of the ultrasound probe, thereby achieving precise positioning of the puncture position and improving the precision and accuracy of puncture guidance.

In some embodiments, determining, based on the sensor coordinates, the first matrix, and/or the second matrix, the second position conversion relationship between the 2D coordinate system and the spatial coordinate system corresponding to the positioning sensor connected to the ultrasound probe includes any one of the following:

The first method is to obtain a first coordinate of the target mapping point in the 2D coordinate system, and determine a second coordinate of the target mapping point in the spatial coordinate system based on the first matrix, the second matrix, and the first coordinate; and establish the second position transformation relationship based on the first coordinate and the second coordinate;

The second method is to obtain vertex coordinates of the vertices of the transverse plane image in the spatial coordinate system based on the first matrix, the second matrix and a size of the transverse plane image; determine the second coordinates of the target mapping point in the spatial coordinate system based on the first coordinates and the vertex coordinates; and establish the second position transformation relationship based on the first coordinates and the second coordinates.

By way of example, the first method of determining the second coordinate based on the first matrix, the second matrix and the first coordinate can be: $P_{E3\_3d} = TM\_sensor \times TM\_trans \times (U_{E3}, V_{E3}, 0, 1)$; wherein, TM_sensor indicates the first matrix; and TM _trans indicates the second matrix; and $U_{E3}, V_{E3}$ indicates the second coordinate of the target mapping point in the 2D coordinate system in the transverse plane image.

By way of example, as shown in FIG.11, which is a schematic diagram of the vertices of a transverse plane image, a calculation method for determining the vertex coordinates of the vertices of the transverse plane image in the spatial coordinate system includes:

$$P_{tl} = TM\_sensor \times TM\_trans \times (0,0,0,1);$$

$$P_{tr} = TM\_sensor \times TM\_trans \times (w\text{-}1,0,0,1);$$

$$P_{bl} = TM\_sensor \times TM\_trans \times (0,h\text{-}1,0,1);$$

$$P_{br} = TM\_sensor \times TM\_trans \times (w\text{-}1,h\text{-}1,0,1);$$

wherein, TM_sensor indicates the first matrix; and TM_trans indicates the second matrix; and w indicates a length of the transverse plane image; and h indicates a width of the transverse plane image; and $P_{tl}$ indicates the coordinates of the upper left vertex in the transverse plane image; and $P_{tr}$ indicates the coordinates of the upper right vertex in the transverse plane image; and $P_{bl}$ indicates the coordinates of the lower left vertex in the transverse plane image; and $P_{br}$ indicates the coordinates of the lower right vertex in the transverse plane image.

By way of example, the second method of determining the second coordinate according to the first coordinate and the vertex coordinate includes:

$$\overrightarrow{vx} = (P_{tr}\text{-}P_{tl}) / \left| P_{tr}\text{-}P_{tl} \right|;$$

$$\overrightarrow{vy} = (P_{bl}\text{-}P_{tl}) / \left| P_{bl}\text{-}P_{tl} \right|;$$

$$P_{E3\_3d} = P_{tl} + \overrightarrow{vx} \times U_{E3} + \overrightarrow{vy} \times V_{E3}$$

wherein, $P_{tl}$ indicates the coordinates of the upper left corner vertex in the transverse plane image; and $P_{tr}$ indicates the coordinates of the upper right corner vertex in the transverse plane image; and $P_{bl}$ indicates the coordinates of the lower left corner vertex in the transverse plane image; and $U_{E3}$, $V_{E3}$ indicate the first coordinates of the target mapping point in the 2D coordinate system in the transverse plane image.

In some embodiments, the two methods of determining the second coordinate in the above embodiments determine the conversion relationship between the first coordinate in the 2D coordinate system and the second coordinate in the spatial coordinate system, thereby establishing the second position conversion relationship. Based on the second position conversion relationship, the spatial position conversion between the two coordinate systems can be realized.

In the embodiment of the present application, the second position conversion relationship between the 2D coordinate system corresponding to the ultrasound image and the spatial coordinate system of the positioning sensor can be accurately established by a variety of methods, so that the ultrasound probe does not need to be moved to the center position after each rotation, simplifying the operation and improving efficiency.

In some embodiments, determining the coordinate data of the target mapping point located in the sagittal plane imaging area in the 2D coordinate system according to the second position conversion relationship includes:

updating the second matrix of the positioning sensor in the spatial coordinate system to a third matrix; wherein, the third matrix is used to represent a third position conversion relationship between the positioning sensor and the rotated ultrasound probe;

based on the second coordinates of the target mapping point located in the sagittal plane imaging area and the third matrix, determining the coordinate data of the target mapping point located in the sagittal plane imaging area in the 2D coordinate system corresponding to the updated transverse plane image by using the second position transformation relationship.

By way of example, a method for updating the first coordinate of the target mapping point located in the imaging area on the sagittal plane in the 2D coordinate system includes:

$$P_{E3\_2d\_new\_temp} = TM\_trans' \times P_{E3\_3d};$$

$$U_{E3\_2d\_new} = P_{E3\_2d\_new\_temp}[0];$$

$$V_{E3\_2d\_new} = P_{E3\_2d\_new\_temp}[1];$$

$$P_{E3\_2d\_new} = (U_{E3\_2d\_new}, V_{E3\_2d\_new});$$

wherein, TM_trans' indicates the third matrix; and $P_{E3\_3d}$ indicates the second coordinate of the target mapping point; and indicates the horizontal coordinate in the first coordinate; and $V_{E3\_2d\_new}$ indicates the vertical coordinate in the first coordinate; and $P_{E3\_2d\_new}$ indicates the first coordinate.

In the embodiment of the present application, after rotating the ultrasound probe, the computer device can update the first coordinate of the target mapping point on the updated transverse plane image by the second position conversion relationship between the 2D coordinate system corresponding to the ultrasound image and the spatial coordinate system corresponding to the positioning sensor connected to the ultrasound probe, while keeping the second coordinate of the target mapping point unchanged, thereby achieving precise positioning of the target mapping point and improving the accuracy and precision of puncture guidance.

In the embodiments of the present application, the following provides specific examples in combination with any of the above embodiments.

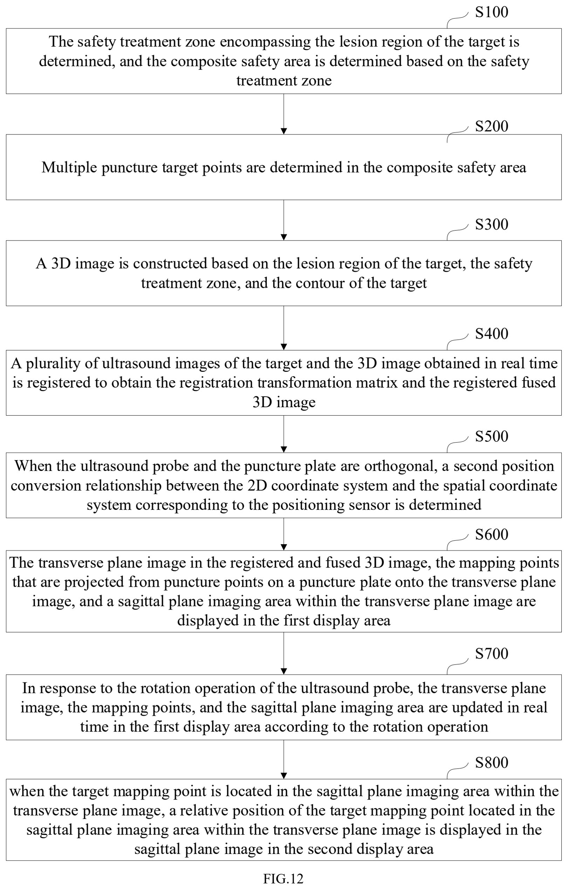

Exemplary Embodiment 1: as shown in FIG.12, which is a flow chart of the computer device implementing puncture guidance; when the processor in the computer device executes computer programs, the following steps are implemented.

S100, the safety treatment zone encompassing the lesion region of the target is determined, and the composite safety area is determined based on the safety treatment zone.

In an optional embodiment, when the lesion region is a 2D area, the computer device inputs multiple frames of 2D images containing the target into the segmentation model to perform lesion region identification and segmentation, and determines the 2D image containing the lesion region, and obtains a preset first distance. Based on the first distance corresponding to each of the contour points of the 2D lesion region, the computer device moves each of the contour points of the 2D lesion region along the corresponding unit normal vector in the opposite direction of the 2D lesion region, thereby obtaining the 2D target point after each of the contour points is moved. The computer device uses the convex hull algorithm to generate a minimum convex polygon corresponding to the 2D image containing the 2D lesion region, and smooth the generated minimum convex polygon to obtain the initial safe region encompassing the 2D lesion region. If the contour of the initial safe region extends beyond the contour of the target, the computer device replaces the contour of the exceeding portion of the initial safe region with the contour of the target, thereby obtaining multiple 2D safety treatment zones. The computer device determines the first frame of the 2D image containing the lesion region as the first frame image, and the last frame of the 2D image containing the lesion region as the last frame image. The computer device generates safety treatment zones in the preceding n 2D image frames adjacent to the first frame based on the shape and area of the safety treatment zone in the first frame. The computer device generates safety treatment zones in the succeeding n 2D image frames adjacent to the last frame based on the shape and area of the safety treatment zone in the last frame. The computer device spatially aligns the 2D images containing the 2D lesion region, the preceding n 2D image frames, and the succeeding n 2D image frames. The computer device converts the 2D contour points of the safety treatment zone in each 2D image frame into the 3D point cloud, assigns the depth information, and performs Delaunay triangulation on the 3D point cloud to generate the triangulated mesh. Based on the triangulated mesh, the computer device slices the 3D safety treatment zone according to a preset slice spacing to obtain multiple 2D safety treatment zones. The multiple 2D safety treatment zones are displayed in the 2D image that includes the 2D lesion region and the 2D target. The computer device uses the U-shaped network to extract the safety treatment zone in each frame of the 2D image, and projects the extracted multiple 2D safety treatment zones onto the same 2D plane to obtain a composite safety area.

In an optional embodiment, when the lesion region is a 3D zone, the computer device obtains the second distance to be moved from the current position of each of the contour points on the surface of the 3D lesion region, and the unit normal vector of each of the contour points on the surface of the 3D lesion region. The computer device moves each of the contour points along the unit normal vector by the corresponding second distance to obtain the target point after each of the contour points is moved. The computer device generates the triangulated mesh based on the position coordinates of each target point by Delaunay triangulation, and constructs the 3D safety treatment zone based on the triangulated mesh. The computer device directly projects the 3D safety treatment zone onto a 2D plane to obtain the composite safety area.

S200, multiple puncture target points are determined in the composite safety area.

In an optional embodiment, the computer device divides the composite safety area into blocks according to the area, the perimeter, or the diameter of the composite safety area to obtain multiple sub-areas. At the same time, the computer device also obtains the ablation distance of the interventional ablation device. In each of the sub-areas, the computer device takes any contour point in the boundary of the sub-area as the first puncture target point, and determines in the sub-area a candidate puncture target point whose distance from the first puncture target point is less than or equal to the ablation distance. The computer device compares the candidate puncture target point with each pre-stored avoidance position. If the candidate puncture target point coincides with one of the avoidance positions, the computer adjusts the candidate puncture target point, and if the adjusted candidate puncture target point does not coincide with any of the avoidance positions, the computer determines the adjusted candidate puncture target point as the next puncture target point, wherein the distance between the adjusted candidate puncture target point and the first puncture target point is less than or equal to the ablation distance, and the replaced candidate puncture target point is also located in the sub-area. When the candidate puncture target point does not coincide with the avoidance position of the target, the computer device directly determines the candidate puncture target point as the next puncture target point until the puncture target points are evenly distributed throughout the sub-areas.

S300, a 3D image is constructed based on the lesion region of the target, the safety treatment zone, and the contour of the target.

S400, the ultrasound image of the target and the 3D image obtained in real time is registered to obtain the registration transformation matrix and the registered fused 3D image.

In an optional embodiment, the registration transformation matrix is used to indicate the spatial conversion relationship between the 2D coordinate system corresponding to the ultrasound image and the 3D coordinate system corresponding to the 3D image.

S500, when the ultrasound probe and the puncture plate are orthogonal, a second position conversion relationship between the 2D coordinate system and the spatial coordinate system corresponding to the positioning sensor is determined.

In an optional embodiment, when the ultrasound probe and the puncture plate are orthogonal, the puncture point on the puncture plate is projected onto the transverse plane image; and the projected mapping point on the transverse plane image can accurately guide the puncture operation of the target. The computer device obtains sensor coordinates, the first matrix and the second matrix of the positioning sensor in the corresponding spatial coordinate system via the positioning sensor connected to the ultrasound probe, wherein the first matrix is a matrix determined based on the sensor coordinate transformation, and the second matrix is used to represent the first position transformation relationship between the positioning sensor and the ultrasound probe. Based on the sensor coordinates, the first matrix and /or the second matrix, the second position transformation relationship between the 2D coordinate system and the spatial coordinate system corresponding to the positioning sensor is determined.

S600, the transverse plane image in the registered and fused 3D image, the mapping points that are projected from puncture points on a puncture plate onto the transverse plane image, and a sagittal plane imaging area within the transverse plane image are displayed in the first display area; wherein each of the plurality of puncture targets corresponds to a target mapping point, the target mapping point indicates the mapping point closest in distance to the corresponding puncture target.

S700, in response to the rotation operation of the ultrasound probe, the transverse plane image, the mapping points, and the sagittal plane imaging area are updated in real time in the first display area according to the rotation operation.

S800, when the target mapping point is located in the sagittal plane imaging area within the transverse plane image, a relative position of the target mapping point located in the sagittal plane imaging area within the transverse plane image is displayed in the sagittal plane image in the second display area; wherein each of the plurality of puncture targets corresponds to a target mapping point, the target mapping point indicates the mapping point closest in distance to the corresponding puncture target.

In an optional embodiment, the target mapping point indicates the mapping point closest to the puncture target point. When the target mapping point of the current puncture is located in the sagittal plane imaging area on the transverse plane image, the rotation of the ultrasound probe can be stopped; and the coordinate data of the target mapping point of the current mapping in the 2D coordinate system is updated by the second position transformation relationship; and the target coordinate data of the target mapping point of the current puncture in the 3D coordinate system is determined based on the registration transformation matrix and the updated coordinate data of the target mapping point of the current mapping; and the relative position of the target mapping point located in the sagittal plane image within the transverse plane image being displayed in the sagittal plane imaging area in the second display area based on the target coordinate data.

The computer device and system for implementing puncture guidance described above determine a safety treatment zone that encompasses the lesion region of the target; and determine multiple puncture target points in the safety treatment zone. When there is a lesion outside the determined lesion region of the target, multiple puncture target points can be determined in the safety treatment zone that encompasses the lesion region to guide the interventional ablation device to perform puncture and/or ablation. Moreover, in response to the rotation operation of the ultrasound probe, information such as the transverse plane image, the mapping points, and the sagittal plane imaging area can be updated in real time during the rotation of the ultrasound probe. When the target mapping point coincides with the sagittal plane imaging area within the transverse plane image, the relative position of the target mapping point located in the sagittal plane imaging area within the transverse plane image can be displayed in the second display area; thereby achieving the same target mapping point being displayed simultaneously in the transverse plane image and the sagittal plane image. On the one hand, the puncture position of each puncture can be accurately determined, with relatively high accuracy and stability, reducing the occurrence of multiple punctures due to inaccurate puncture positions; on the other hand, there is no need to repeatedly move the ultrasound probe to observe the puncture position for each puncture. After the last puncture, the ultrasound probe can be directly rotated to the next target mapping point for puncture, which simplifies the operation, saves time and improves puncture efficiency.

It should be understood that, although the various steps in the flowcharts involved in the various embodiments described above are displayed in sequence according to the instructions of the arrows, these steps are not necessarily executed in sequence in the order indicated by the arrows. Unless otherwise specified herein, there is no strict order restriction on the execution of these steps, and these steps can be executed in other orders. Moreover, at least a portion of the steps in the flowcharts involved in the various embodiments described above can include multiple steps or multiple stages, and these steps or stages are not necessarily executed and completed at the same time, but can be executed at different times, and the execution order of these steps or stages is not necessarily to be carried out in sequence, but can be executed in turn or alternately with other steps or at least a portion of steps or stages in other steps.

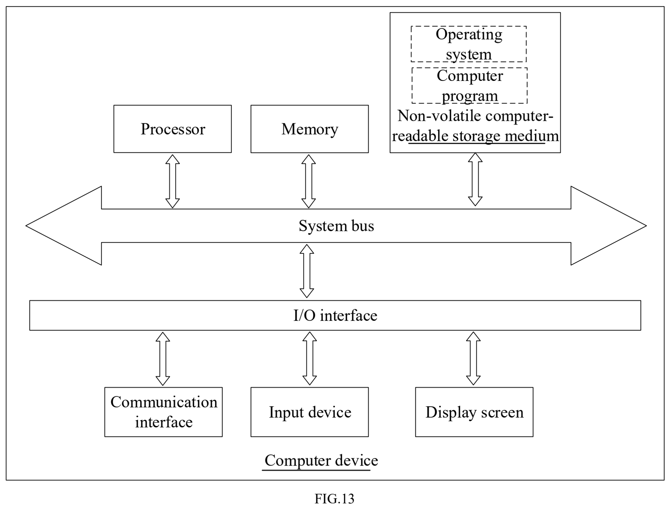

In one embodiment, a computer device is provided. The computer device may be a terminal, and its internal structure may be as shown in FIG.13. The computer device includes a processor, a memory, a communication interface, a display screen, and an input device connected via a system bus. The processor of the computer device is used to provide computing and control capabilities. The memory of the computer device includes a non-volatile storage medium and an internal memory. The non-volatile storage medium stores an operating system and a computer program. The internal memory provides an environment for the operation of the operating system and computer program in the non-volatile storage medium. The communication interface of the computer device is used to communicate with an external terminal via wired or wireless communication. The wireless communication may be achieved via Wi-Fi, a mobile cellular network, NFC (near-field communication), or other technologies. When executed by the processor, the computer program implements a method for detecting the safety of aerial work. The display screen of the computer device may be a liquid crystal display or an electronic ink display. The input device of the computer device may be a touch layer covering the display screen, or may be a key, trackball, or touchpad provided on the computer device housing, or may be an external keyboard, touchpad, or mouse.

Those skilled in the art will understand that the structure shown in FIG.13 is merely a block diagram of a portion of the structure related to the solution of the present application, and does not constitute a limitation on the computer device to which the solution of the present application is applied. The specific computer device may include more or fewer components than shown in the figure, or combine certain components, or have a different arrangement of components.

In one embodiment, a computer-readable storage medium is provided, on which a computer program is stored. When the computer program is executed by a processor, the steps in the above-mentioned method embodiments are implemented.

In one embodiment, a computer program product is provided, comprising a computer program, which implements the steps performed by a processor of a computer device when the computer program is executed by a processor.

It should be noted that the user information (including but not limited to user device information, user personal information, etc.) and data (including but not limited to data used for analysis, stored data, displayed data, etc.) involved in the application are all information and data authorized by the user or fully authorized by all parties.

Those skilled in the art will appreciate that all or part of the processes in the above-described method embodiments can be implemented by instructing the relevant hardware through a computer program. The computer program can be stored in a non-volatile computer-readable storage medium. When executed, the computer program can include the processes of the above-described method embodiments. Any reference to a memory, database, or other medium used in the embodiments provided herein may include at least one of non-volatile and volatile memory. Non-volatile memory may include a read-only memory (ROM), a magnetic tape, a floppy disk, a flash memory, an optical memory, a high-density embedded non-volatile memory, a resistive random access memory (ReRAM), a magnetic random access memory (MRAM), a ferroelectric random access memory (FRAM), a phase change memory (PCM), a graphene memory, etc. Volatile memory may include random access memory (RAM) or external cache memory, etc. By way of illustration and not limitation, RAM can take various forms, such as a static random access memory (SRAM) or a dynamic random access memory (DRAM). The databases involved in the various embodiments provided herein may include at least one of a relational database and a non-relational database. Non-relational databases may include, but are not limited to, distributed databases based on blockchains. The processors involved in the various embodiments provided herein may be, but are not limited to, general-purpose processors, central processing units (CPUs), graphics processing units (GPUs), digital signal processors (DSPs), programmable logic devices (PLDs), data processing logic devices based on quantum computing, and the like.

The technical features of the above embodiments can be combined arbitrarily. To make the description concise, not all possible combinations of the technical features in the above embodiments are described. However, as long as there is no contradiction in the combination of these technical features, they should be considered to be within the scope of this specification.

The above-described embodiments merely represent several implementation methods of the present application. While the descriptions are relatively specific and detailed, they should not be construed as limiting the scope of the present application. It should be noted that a person of ordinary skill in the art may make various modifications and improvements without departing from the spirit of the present application, and these modifications and improvements fall within the scope of protection of the present application. Therefore, the scope of protection of the present application shall be determined by the appended claims.

**Claims**

1.    A computer device for implementing puncture guidance, **characterized in that** the computer device comprises a memory and a processor, and the memory stores a computer program, and the processor is configured to execute the computer program to implement the following steps: :

determining a safety treatment zone encompassing a lesion region of a target;
determining a plurality of puncture target points in the safety treatment zone;
constructing a 3D image based on the lesion region of the target, the safety treatment zone, and a contour of the target;
registering a plurality of ultrasound images of the target obtained in real time with the 3D image to obtain a registration transformation matrix and a registered and fused 3D image; wherein the plurality of ultrasound images is obtained by an ultrasound probe, and the registration transformation matrix is used to indicate a spatial conversion relationship between a 3D coordinate system corresponding to the 3D image and a 2D coordinate system corresponding to the ultrasound image;
in the first display area, displaying a transverse plane image in the registered and fused 3D image, mapping points that are projected from puncture points on a puncture plate onto the transverse plane image, and a sagittal plane imaging area within the transverse plane image; wherein each of the plurality of puncture targets corresponds to a target mapping point, the target mapping point indicates the mapping point closest in distance to the corresponding puncture target;
in response to a rotation operation of the ultrasound probe, updating the transverse plane image, the mapping points, and the sagittal plane imaging area in the first display area in real time according to the rotation operation; when the target mapping point is located in the sagittal plane imaging area within the transverse plane image, a relative position of the target mapping point located in the sagittal plane imaging area within the transverse plane image being displayed in the sagittal plane image in the second display area.

2.    The computer device according to claim 1, wherein the lesion region is a 2D lesion region; and determining the safety treatment zone encompassing the lesion region of the target comprises:

determining, in multiple frames of 2D images of the target, a 2D image containing the 2D lesion region, and obtaining a preset first distance;
in the 2D image containing the 2D lesion region, generating an initial safety zone encompassing the 2D lesion region based on the preset first distance;
when a contour of the initial safety zone extends beyond the contour of the target, replacing the contour of an exceeding portion of the initial safety zone with the contour of the target to obtain a plurality of 2D safety treatment zones;
generating a 3D safety treatment zone based on the plurality of 2D safety treatment zones.

3.    The computer device according to claim 1, wherein the lesion region is a 3D lesion region; and determining the safety treatment zone encompassing the lesion region of the target comprises:

obtaining a unit normal vector of each of contour points on a surface of the 3D lesion region, and a second distance that each of the contour points moves along the unit normal vector;

moving each of the contour points along the unit normal vector by the second distance to obtain target points after the contour points are moved;

based on the target points, obtaining the safety treatment zone encompassing the 3D lesion region of the target.

4. The computer device according to claim 3, wherein obtaining the second distance that each of the contour points moves along the unit normal vector comprises:

presetting an initial movement distance for each of the contour points along the unit normal vector;

respectively determining a temporary point after each of the contour points moves along corresponding unit normal vector based on the initial movement distance, 3D coordinates of each of the contour points, and the unit normal vector of each of the contour points;

determining a target ray formed by the 3D coordinates of the temporary point and the unit normal vector of the contour point;

when the number of intersection points between the target ray and the contour of the target is even, adjusting the initial movement distance until the number of intersection points between the target ray and the contour of the target is odd, and obtaining the second distance corresponding to each of the contour points.

5. The computer device according to claim 1, wherein determining the plurality of puncture target points in the safety treatment zone comprises:

determining a composite safety area for the lesion region of the target according to the safety treatment zone, and the composite safety area being a 2D area;

according to size information of the composite safety area, dividing the composite safety area into blocks to obtain a plurality of sub-areas, and obtaining an ablation distance of an interventional ablation device;

in a contour of each of the plurality of sub-areas, respectively determining any contour point of each of the plurality of sub-areas as a first puncture target point, and determining a next puncture target point according to the ablation distance and a position of the first puncture target point, until the plurality of puncture target points are evenly distributed throughout each of the plurality of sub-areas.

6. The computer device according to claim 5, wherein, in the contour of each of the plurality of sub-areas, respectively determining any contour point of each of the plurality of sub-areas as the first puncture target point, and determining the next puncture target point according to the ablation distance and the position of the first puncture target point, until the plurality of puncture target points are evenly distributed throughout each of the plurality of sub-areas, comprising :

in each of the plurality of sub-areas, determining any contour point of each of the plurality of sub-areas as the first puncture target point;

determining a candidate puncture target point in each of the plurality of sub-areas based on the position of the first puncture target point and the ablation distance; wherein a distance between the candidate puncture target point and the first puncture target point is less than or equal to the ablation distance;

when the candidate puncture target point coincides with an avoidance position of the target, adjusting the candidate puncture target point until the adjusted candidate puncture target point no longer coincides with the avoidance position, and determining adjusted candidate puncture target point as the next puncture target point.

7. The computer device according to claim 1, wherein the processor being further configured to execute the computer program to:

when the ultrasound probe and the puncture plate are orthogonal, obtain sensor coordinates, a first matrix and a second matrix of a positioning sensor in a spatial coordinate system by a positioning sensor connected to the ultrasound probe; wherein the first matrix is a matrix determined according to a sensor coordinate transformation, and the second matrix is used to represent a first position transformation relationship between the positioning sensor and the ultrasound probe;

according to the sensor coordinates, the first matrix, or the second matrix, determine a second position conversion relationship between the 2D coordinate system and the spatial coordinate system corresponding to the positioning sensor connected to the ultrasound probe;

when the target mapping point is located in the sagittal plane imaging area within the transverse plane image, a relative position of the target mapping point located in the sagittal plane imaging area within the transverse plane image being displayed in the sagittal plane image in the second display area, comprises:

determining coordinate data of the target mapping point located in the sagittal plane imaging area in the 2D coordinate system according to the second position conversion relationship;
determining target coordinate data of the target mapping point located in the sagittal plane imaging area in the 3D coordinate system according to the coordinate data and the registration transformation matrix;
in the second display area, the relative position of the target mapping point located in the sagittal plane image within the transverse plane image being displayed in the sagittal plane imaging area according to the target coordinate data.

8. The computer device according to claim 7, wherein, according to the sensor coordinates, the first matrix, or the second matrix, determining the second position conversion relationship between the 2D coordinate system and the spatial coordinate system corresponding to the positioning sensor connected to the ultrasound probe comprises:

obtaining a first coordinate of the target mapping point in the 2D coordinate system, and determining a second coordinate of the target mapping point in the spatial coordinate system based on the first matrix, the second matrix, and the first coordinate; and establishing a second position transformation relationship based on the first coordinate and the second coordinate; or
obtaining vertex coordinates of vertices of the transverse plane image in the spatial coordinate system based on the first matrix, the second matrix and a size of the transverse plane image; and determining second coordinates of the target mapping point in the spatial coordinate system based on the first coordinates and the vertex coordinates; and establishing the second position transformation relationship based on the first coordinates and the second coordinates.

9. The computer device according to claim 8, wherein determining the coordinate data of the target mapping point located in the sagittal plane imaging area in the 2D coordinate system according to the second position conversion relationship comprises:

updating the second matrix of the positioning sensor in the spatial coordinate system to a third matrix; wherein the third matrix is used to represent a third position conversion relationship between the positioning sensor and a rotated ultrasound probe;
based on the second coordinates of the target mapping point located in the sagittal plane imaging area and the third matrix, determining the coordinate data of the target mapping point located in the sagittal plane imaging area in the 2D coordinate system corresponding to the updated transverse plane image by using the second position transformation relationship.

10. A puncture guidance system, **characterized in that** the system comprises a computer device according to any one of claims 1 to 9, an ultrasound probe, an interventional ablation device, a puncture plate, and a navigation unit; and the navigation unit comprises a positioning sensor; the ultrasound probe, the interventional ablation device, and the navigation unit are all communicatively connected to the computer device.

11. A method for implementing puncture guidance, **characterized in that** the method comprises:

determining a safety treatment zone encompassing a lesion region of a target;
determining a plurality of puncture target points in the safety treatment zone;
constructing a 3D image based on the lesion region of the target, the safety treatment zone, and a contour of the target;
registering a plurality of ultrasound images obtained in real time with the 3D image to obtain a registration transformation matrix and a registered and fused 3D image; wherein the plurality of ultrasound images is obtained by an ultrasound probe, and the registration transformation matrix is used to indicate a spatial conversion relationship between a 3D coordinate system corresponding to the 3D image and a 2D coordinate system corresponding to the ultrasound image;
in the first display area, displaying a transverse plane image in the registered and fused 3D image, mapping points that are projected from puncture points on a puncture plate onto the transverse plane image, and a sagittal plane imaging area within the transverse plane image; wherein each of the plurality of puncture targets corresponds to a target mapping point, the target mapping point indicates the mapping point closest in distance to the corresponding puncture target;

in response to a rotation operation of the ultrasound probe, updating the transverse plane image, the mapping points, and the sagittal plane imaging area in the first display area in real time according to the rotation operation;

when the target mapping point is located in the sagittal plane imaging area within the transverse plane image, a relative position of the target mapping point located in the sagittal plane imaging area within the transverse plane image being displayed in the sagittal plane image in the second display area.

12. The method according to claim 11, wherein the lesion region is a 2D lesion region; and determining the safety treatment zone encompassing the lesion region of the target comprises:

determining, in multiple frames of 2D images of the target, a 2D image containing the 2D lesion region, and obtaining a preset first distance;

in the 2D image containing the 2D lesion region, generating an initial safety zone encompassing the 2D lesion region based on the preset first distance;

when a contour of the initial safety zone extends beyond the contour of the target, replacing the contour of an exceeding portion of the initial safety zone with the contour of the target to obtain a plurality of 2D safety treatment zones;

generating a 3D safety treatment zone based on the plurality of 2D safety treatment zones.

13. The method according to claim 11, wherein the lesion region is a 3D lesion region; and determining the safety treatment zone encompassing the lesion region of the target comprises:

obtaining a unit normal vector of each of contour points on a surface of the 3D lesion region, and a second distance that each of the contour points moves along the unit normal vector;

moving each of the contour points along the unit normal vector by the second distance to obtain target points after the contour points are moved;

based on the target points, obtaining the safety treatment zone encompassing the 3D lesion region of the target.

14. The method according to claim 13, wherein obtaining the second distance that each of the contour points moves along the unit normal vector comprises:

presetting an initial movement distance for each of the contour points along the unit normal vector;

respectively determining a temporary point after each of the contour points moves along corresponding unit normal vector based on the initial movement distance, 3D coordinates of each of the contour points, and the unit normal vector of each of the contour points;

determining a target ray formed by the 3D coordinates of the temporary point and the unit normal vector of the contour point;

when the number of intersection points between the target ray and the contour of the target is even, adjusting the initial movement distance until the number of intersection points between the target ray and the contour of the target is odd, and obtaining the second distance corresponding to each of the contour points.

15. The method according to claim 11, wherein determining the plurality of puncture target points in the safety treatment zone comprises:

determining a composite safety area for the lesion region of the target according to the safety treatment zone, and the composite safety area being a 2D area;

according to size information of the composite safety area, dividing the composite safety area into blocks to obtain a plurality of sub-areas, and obtaining an ablation distance of an interventional ablation device;

in a contour of each of the plurality of sub-areas, respectively determining any contour point of each of the plurality of sub-areas as a first puncture target point, and determining a next puncture target point according to the ablation distance and a position of the first puncture target point, until the plurality of puncture target points are evenly distributed throughout each of the plurality of sub-areas.

103

Interventional
ablation device

102

Biplane Ultrasound
probe

101

Computer device

105

Navigation unit

104

Puncture plate

FIG.1

S201

A safety treatment zone encompassing a lesion region of a target is determined

S202

A plurality of puncture target points are determined in the safety treatment zone

S203

A 3D image is constructed based on the lesion region of the target, the safety treatment zone, and the contour of the target

S204

A plurality of ultrasound images of the target obtained in real time is registered with the 3D image to obtain a registration transformation matrix and a registered and fused 3D image; wherein the plurality of ultrasound images is obtained by an ultrasound probe, and the registration transformation matrix is used to indicate a spatial conversion relationship between the 3D coordinate system corresponding to the 3D image and the 2D coordinate system corresponding to the ultrasound image

S205

In a first display area, the transverse plane image in the registered and fused 3D image, mapping points that are projected from puncture points on a puncture plate onto the transverse plane image, and a sagittal plane imaging area within the transverse plane image are displayed

S206

In response to a rotation operation of the ultrasound probe, the transverse plane image, the mapping points, and the sagittal plane imaging area are updated in the first display area in real time according to the rotation operation

S207

when the target mapping point is located in the sagittal plane imaging area within the transverse plane image, a relative position of the target mapping point located in the sagittal plane imaging area within the transverse plane image is displayed in the sagittal plane image in the second display area

FIG.2

FIG.3

FIG.4

First display area

Second display area

FIG.5

| | Contour of the initial zone | | A part |
| --- | --- | --- | --- |
| | Contour of the target | | B part |
| | Lesion region | | |

FIG.6

| | 2D safety treatment zone | | Contour of the target |
| --- | --- | --- | --- |
| | 2D Lesion region | | |

FIG.7

| | Overlapping area | | Sub-area |
| --- | --- | --- | --- |

FIG.8

FIG.9

FIG.10

FIG.11

S100

The safety treatment zone encompassing the lesion region of the target is determined, and the composite safety area is determined based on the safety treatment zone

S200

Multiple puncture target points are determined in the composite safety area

S300

A 3D image is constructed based on the lesion region of the target, the safety treatment zone, and the contour of the target

S400

A plurality of ultrasound images of the target and the 3D image obtained in real time is registered to obtain the registration transformation matrix and the registered fused 3D image

S500

When the ultrasound probe and the puncture plate are orthogonal, a second position conversion relationship between the 2D coordinate system and the spatial coordinate system corresponding to the positioning sensor is determined

S600

The transverse plane image in the registered and fused 3D image, the mapping points that are projected from puncture points on a puncture plate onto the transverse plane image, and a sagittal plane imaging area within the transverse plane image are displayed in the first display area

S700

In response to the rotation operation of the ultrasound probe, the transverse plane image, the mapping points, and the sagittal plane imaging area are updated in real time in the first display area according to the rotation operation

S800

when the target mapping point is located in the sagittal plane imaging area within the transverse plane image, a relative position of the target mapping point located in the sagittal plane imaging area within the transverse plane image is displayed in the sagittal plane image in the second display area

FIG.12

FIG.13

Europäisches
Patentamt

European
Patent Office

Office européen
des brevets

**EUROPEAN SEARCH REPORT**

**Application Number**

EP 25 21 7629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 118 021 410 A (KABEN SHENZHEN MEDICAL DEVICES CO LTD ET AL.) 14 May 2024 (2024-05-14) * paragraphs [0005] - [0007], [0013] - [0015], [0022], [0026] - [0028], [0068] - [0070]; claims; figures * | 1-15 | INV. A61B8/08 A61B8/12 A61B8/00 |
| X,P | CN 119 184 819 A (KABEN SHENZHEN MEDICAL DEVICES CO LTD) 27 December 2024 (2024-12-27) | 1,10,11 | |
| A,P | * paragraphs [0005], [0009] - [0012], [0020] - [0027], [0031] - [0046], [0055] - [0071], [0092] - [0095]; claims; figures * | 2-9, 12-15 | |
| X,P | CN 119 214 759 A (KABEN SHENZHEN MEDICAL DEVICES CO LTD) 31 December 2024 (2024-12-31) | 1,10,11 | |
| A,P | * paragraphs [0004] - [0016]; claims; figures * | 2-9, 12-15 | |

**TECHNICAL FIELDS SEARCHED (IPC)**

A61B

The present search report has been drawn up for all claims

| Place of search | Date of completion of the search | Examiner |
|---|---|---|
| Munich | 27 March 2026 | Mundakapadam, S |

EPO FORM 1503 03.82 (P4C01)

## ANNEX TO THE EUROPEAN SEARCH REPORT
## ON EUROPEAN PATENT APPLICATION NO.

EP 25 21 7629

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2026

| Patent document cited in search report | | Publication date | Patent family member(s) | | Publication date |
|---|---|---|---|---|---|
| CN 118021410 | A | 14-05-2024 | CN | 118021410 A | 14-05-2024 |
| | | | EP | 4631438 A1 | 15-10-2025 |
| | | | JP | 2025161763 A | 24-10-2025 |
| | | | NL | 4000050 A | 03-11-2025 |
| | | | US | 2025318814 A1 | 16-10-2025 |
| CN 119184819 | A | 27-12-2024 | NONE | | |
| CN 119214759 | A | 31-12-2024 | NONE | | |

EPO FORM P0459

For more details about this annex : see Official Journal of the European Patent Office, No. 12/82